(19) Europäisches Patentamt
European Patent Office
Office européen des brevets

(11) EP 4 057 003 A1

(12) EUROPEAN PATENT APPLICATION

(43) Date of publication:
**14.09.2022 Bulletin 2022/37**

(21) Application number: **21382203.4**

(22) Date of filing: **12.03.2021**

(51) International Patent Classification (IPC):
***G01N 33/52*** *(2006.01)* ***G01N 33/84*** *(2006.01)*

(52) Cooperative Patent Classification (CPC):
**G01N 33/52; G01N 33/84**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Universidad de Castilla la Mancha**
**13071 Ciudad Real (ES)**

(72) Inventors:
- **BRAVO PEREZ, Ivan**
**02002 Albacete (ES)**
- **ALONSO MORENO, Carlos**
**02002 Albacete (ES)**
- **CARRILLO HERMOSILLA, Fernando**
**13003 Ciudad Real (ES)**
- **PACHECO LIÑAN, Pedro José**
**23265 Jaén (ES)**

(74) Representative: **Balder IP Law, S.L.**
**Paseo de la Castellana 93**
**5ª planta**
**28046 Madrid (ES)**

# (54) SELECTIVE SULFATE ANION DETECTION USING GUANYLATED FLUOROPHORES

(57) The present invention relates to the use of a guanylated fluorphore as a sulfate sensor and more in particular it relates to a method for selectively detecting and, optionally, quantifying sulfate anions in an aqueous sample by fluorescence spectroscopy using a guanylated fluorophore comprising a fluorescent group, containing a substituted or unsubstituted planar fluorescent core, and a guanidinyl moiety. The present invention also relates to a guanylated fluorophore comprising a fluorescent group and a guanidinyl moiety, wherein the fluorescent group is a fluorenyl group and the guanidinyl moiety is selected from an unsubstituted guanidinyl group, an alkyl substituted guanidinyl group and an aryl substituted guanidinyl group. The present invention also relates to method for preparing such a guanylated fluorophore.

EP 4 057 003 A1

## Description

## TECHNICAL FIELD

The invention relates to a method for detecting and quantifying sulfate anions in aqueous samples. The method allows selective detection of sulfate anion, in particular, across a wide pH range.

Therefore, the present invention can be included within the technical field of sulfate sensors and fluorescent sulfate sensors in particular, since the methods described herein use a guanylated fluorophore (derivate guanidine molecules) that act as selective sulfate fluorescence sensors in water which have been found able to function with high sensitivity, in particular, across a wide pH range.

## BACKGROUND OF THE INVENTION

Sensitive and selective detection of anions is an important and active research topic. Since anions are ubiquitous in nature due to their significant role in many biological and chemical processes, their quantity and impact on human health and the environment must be controlled (Y. Zhang and P. S. Cremer, Annu. Rev. Phys. Chem., 2010, 61, 63-83). Among various important anions, sulfate anion ($[SO_4]^{2-}$) plays an important role in biological, technical and environmental processes (I. Ravikumar and P. Ghosh, Chem. Soc. Rev., 2012, 41, 3077). For instance, it is very well known the role of this anion in the metabolism (I. Ravikumar and P. Ghosh, Chem. Soc. Rev., 2012, 41, 3077), and hence, any sulfate unbalance in the environment or the body will affect the biosynthesis causing several diseases like skin and eyes irritation, dehydration or gastrointestinal disorders (A. Singh and M. Agrawal, J. Environ. Biol., 2008, 29, 15-24). High concentration of sulfate in drinking water has a laxative effect that can lead to dehydration which is of special concern for infants (L. C. Backer, Crit. Rev. Clin. Lab. Sci., 2000, 37, 389-400). In addition to that, the use of the sulfate anion as food additive is not restricted. It is used as acidity regulator in the form of, e.g., potassium sulfate (E515), calcium sulfate (E516), ammonium sulfate (E517), and aluminium sulfate (E520). Although the dietary intake of this anion does not represent a risk for healthy people, its increasing use as food additive and presence in drinking water make its accurate recognition, separation and quantification essential (P. A. Gale and C. Caltagirone, Coord. Chem. Rev., 2018, 354, 2-27; K. Ariga, H. Ito, J. P. Hill and H. Tsukube, Chem. Soc. Rev., 2012, 41, 58).

High levels of sulfate ions cause severe environmental problems, especially in the mining, metallurgical, chemical, petrochemical and agricultural sectors. Tailings (piles or dumps) from coal and some metal-bearing ores (especially those rich in pyrite and chalcopyrite) are readily oxidized by water and oxygen, resulting in acid drainage. Such liquid effluents or AMDs (acid mine drainages) contain high levels of heavy metal ions, sulfate ions and acidity and constitute one of the main challenges in the mining industry.

On one hand, high levels of sulfate anions pose major concerns at an industrial level, such as the scaling of pipes and corrosion. In particular, sulfate ions are some of the main contributors to so-called water "mineralization", thereby increasing the conductivity and corrosion potential of receptor bodies. These anions promote, among other adverse effects, the following: corrosion and scaling in pipes, structures and equipment; fouling and deposition in boilers; acidification of soils; and blockage of soil pores, retarding irrigation or water drainage. Sulfate anions may also engender technical problems by, e.g., interfering with vitrification processes (H. Zhou, Y. Zhao, G. Gao, S. Li, J. Lan and J. You, J. Am. Chem. Soc., 2013, 135, 14908-14911; Q. Li, Y. Yue, Y. Guo and S. Shao, Sensors Actuators B Chem., 2012, 173, 797-801). High concentrations of sulfate and thiosulfate ions in process water may also cause significant problems in the flotation of sulfide ores through changing the pulp chemistry, surface chemistry of sulfide minerals and froth stability. In addition to that, formation of colloidal gypsum and scaling problems in process plants are observed when lime is used for pH regulation. Therefore, reducing sulfate and thiosulfate ion concentration in process water is required for minimizing gypsum formation and stabilizing pulp chemistry.

On the other hand, the removal of sulfate ions from waste water also represents an environmental challenge faced by several industrial sectors, such as the mining, metallurgical, chemical and petrochemical industries (W. A. M. Fernando, I.M.S.K. Ilankoon, T. H. Syed, and M.Yellishetty, Minerals Engineering. 2018, 117, 74-90).

A widely used method for the detection and quantification of the sulfate anion is the use of barium chloride, e.g., for the precipitation of sulfate whereby the amount of sulfate is determined by the quantification of the barium salt precipitation by gravimetric analysis or by titration with EDTA. Other methods using barium chloride are described in, for instance, German patent application published as DD295919A which describes an automated measurement of the sulfate content in water containing, based on X-ray fluorescence measurements of Barium ions in order to determine the sulfate content in sulfate-containing water indirectly and stoichiometrically.

Other methodologies to quantify the sulfate anion rely on the use of supramolecular chemistry to pursue optical anion receptors capable of recognizing and sensing anions, which is one of the most successful strategies (P. A. Gale, Chem. Commun., 2011, 47, 82-86; L. Chen, S. N. Berry, X. Wu, E. N. W. Howe and P. A. Gale, Chem, 2020, 6, 61-141; M. J. Langton, C. J. Serpell and P. D. Beer, Angew. Chemie Int. Ed., 2016, 55, 1974-1987). The underlying principle is

linked to the modification of optical receptor response in the presence of the anion in the media, which brings out high sensitivity/selectivity, fast response time and good stability of the designed sensor (C. McDonagh, C. S. Burke and B. D. MacCraith, Chem. Rev., 2008, 108, 400-422).

Nevertheless, current sulfate receptors still have certain problems to tackle, including the simultaneously recognition of other anions such as phosphate, halides or carbonates, which compromises their selectivity. Additionally, the high hydration energy in water and hydrophilicity of the sulfate anion complicates its selectivity and affinity for synthetic receptors (S. Kubik, Chem. Soc. Rev., 2010, 39, 3648; E. A. Katayev, Y. A. Ustynyuk and J. L. Sessler, Coord. Chem. Rev., 2006, 250, 3004-3037).

Thus, there is a need for sulfate sensors which are selective to sulfate anion over other anions which may also be present in aqueous samples. There is also a need for sensors with an acceptable degree of sensitivity in order to detect and quantify sulfate anion when present in small amounts.

Another important parameter to be considered is the pH, as changes in pH may affect the selectivity and sensitivity of the sensors. Thus, there is also a need for a sulfate sensor which is able to detect and quantify sulfate anion at a wide range of pH.

The guanidinyl moiety (also referred to as guanidium moiety) stands out as a cation-subunit due to its Y-way chemical structure, which can bind with a variety of anions, including phosphates, phosphonates, sulfates, sulfonates, and pyrophosphates.

In particular, guanidine or guanidinyl derivatives have been applied in a wide range of biophysical and industrial application such as therapeutic agents, DNA intercalating agents, ion recognition species, proton sponges or polymer backbones in the design of new ion exchange polymer (K. A. Schug and W. Lindner, Chem. Rev., 2005, 105, 67-114; C. Alonso-Moreno, A. Antiñolo, F. Carrillo-Hermosilla and A. Otero, Chem. Soc. Rev., 2014, 43, 3406-3425; V. Raab, J. Kipke, R. M. Gschwind and J. Sundermeyer, Chem. - A Eur. J., 2002, 8, 1682-1693; J. Ran, L. Wu, Y. He, Z. Yang, Y. Wang, C. Jiang, L. Ge, E. Bakangura and T. Xu, J. Memb. Sci., 2017, 522, 267-291; P. J. Pacheco-Liñán, J. Fernandez-Sainz, I. Bravo, A. Garzón-Ruiz, C. Alonso-Moreno, F. Carrillo-Hermosilla, A. Antiñolo and J. Albaladejo, J. Phys. Chem. C, 2018, 122, 9363-9373; E. Bindewald, R. Lorenz, O. Hübner, D. Brox, D.-P. Herten, E. Kaifer and H.-J. Himmel, Dalt. Trans., 2015, 44, 3467-3485).

It has been recently demonstrated that the inclusion of guanidine moieties to fluorescent systems (e.g., naphthene) gives special photophysical features in aqueous solutions, such as non-conventional phenomena like excited-state proton transfer (ESPT), photoinduced electron transfer (PET), or aggregation-induced emission (AIE), although they are described to be strongly dependent on the solvent environment and the pH (P. J. Pacheco-Liñán, J. Fernandez-Sainz, I. Bravo, A. Garzón-Ruiz, C. Alonso-Moreno, F. Carrillo-Hermosilla, A. Antiñolo and J. Albaladejo, J. Phys. Chem. C, 2018, 122, 9363-9373; P. J. J. Pacheco Liñán, C. Martin, C. Alonso-Moreno, A. Juan, D. Hermida Merino, A. Garzon, J. Albaladejo, M. Van der Auweraer, B. Cohen and I. Bravo, Chem. Commun., DOI:10.1039/D0CC00990C).

Guanidines can be synthetized by catalytic methods and easily incorporated into fluorescent systems. Despite of this, only a few examples of fluorescence systems with guanidine group moieties can be found in literature (P. J. Pacheco-Liñán, J. Fernandez-Sainz, I. Bravo, A. Garzón-Ruiz, C. Alonso-Moreno, F. Carrillo-Hermosilla, A. Antiñolo and J. Albaladejo, J. Phys. Chem. C, 2018, 122, 9363-9373; E. Bindewald, R. Lorenz, O. Hübner, D. Brox, D.-P. Herten, E. Kaifer and H.-J. Himmel, Dalt. Trans., 2015, 44, 3467-3485; P. J. J. Pacheco Liñán, C. Martin, C. Alonso-Moreno, A. Juan, D. Hermida Merino, A. Garzon, J. Albaladejo, M. Van der Auweraer, B. Cohen and I. Bravo, Chem. Commun., DOI:10.1039/D0CC00990C; J. Zhou, H. Liu, B. Jin, X. Liu, H. Fu and D. Shangguan, J. Mater. Chem. C, 2013, 1, 4427; M. P. Coles, P. J. Aragón-Sáez, S. H. Oakley, P. B. Hitchcock, M. G. Davidson, Z. B. Maksic, R. Vianello, I. Leito, I. Kaljurand and D. C. Apperley, J. Am. Chem. Soc., 2009, 131, 16858-16868). Furthermore, their use as sulfate sensor is scarce at present. For instance, Katayev *et al.* review the field of anion recognition from the perspective of tetrahedral oxyanion recognition (including phosphate, perchlorate and sulfate anions among other) with a section devoted to guanidinium-type based receptors (E. A. Katayev, Y. A. Ustynyuk and J. L. Sessler, Coord. Chem. Rev., 2006, 250, 3004-3037) and Kobiro *et al.* describe a chiral chromophoric host, possessing a 4-(N,N-dimethylamino)benzoate (DMAB) group tethered to a chiral bicyclic guanidinium subunit, synthesized and applied to the probe for sulfate anion (K. Kobiro and Y. Inoue, J. Am. Chem. Soc., 2003, 125, 421-427). However, such publications do not show the suitability of guanidinium modified fluorophores for the detection and quantification of sulfate in water samples, let alone in the presence of anions other than sulfate or in the presence of different types of counter cations.

## SUMMARY OF THE INVENTION

It has now been found that a guanylated fluorophore comprising a fluorescent group, containing a substituted or an unsubstituted planar fluorescent core, and a guanidinyl moiety can be used for selectively detecting and, optionally, quantifying sulfate anions in aqueous samples by fluorescence spectroscopy. In particular, guanylated fluorophores as described herein have been found to act selectively as sulfate sensors, being able to detect and quantify sulfate anions with an increased sensitivity and selectivity when compared to known methods. Additionally, such increased selectivity

and sensitivity have been found to be maintained across a wide pH-range.

Accordingly, the present invention relates to the use of such a guanylated fluorphore as a sulfate sensor and, more in particular, it relates to a method for selectively detecting and, optionally, quantifying sulfate anions in an aqueous sample by fluorescence spectroscopy using a guanylated fluorophore comprising a fluorescent group, containing a substituted or unsubstituted planar fluorescent core, and a guanidinyl moiety, wherein

- the fluorescent group is selected from: a fluorenyl group, a naphthyl group, an anthracenyl group, a naphthalimidyl group, a coumarinyl group, each being substituted or unsubstituted; and
- the guanidinyl moiety is selected from an alkyl substituted guanidinyl group, an unsubstituted guanidinyl group, and an aryl substituted guanidinyl group.

The present invention also relates to a guanylated fluorophore comprising a fluorescent group and a guanidinyl moiety, wherein the fluorescent group is a fluorenyl group and the guanidinyl moiety is selected from an unsubstituted guanidinyl group, an alkyl substituted guanidinyl group and an aryl substituted guanidinyl group. The present invention also relates to method for preparing such a guanylated fluorophore, comprising: guanylating a mono-amine containing a fluorene group, by reacting the mono-amine containing the fluorene group with *N,N'*-diisopropyl-carbodiimide, in the presence of a catalyst, in particular, selected from $ZnEt_2$, $^{n}BuLi$, $MgBu_2$, trispentafluorophenylborane, and $Zn(OTf_2)$.

BRIEF DESCRIPTION OF THE FIGURES

Figure 1 shows $^{1}H$-NMR in $CDCl_3$ of guanylated fluorophore (A) as prepared in Example 1.

Figure 2 shows $^{13}C$-NMR in $CDCl_3$ of guanylated fluorophore (A) as prepared in Example 1.

Figure 3 shows IR spectrum of guanylated fluorophore (A) as prepared in Example 1.

Figure 4a shows the absorption spectra of guanylated fluorophore (A) at different pH values and Figure 4b shows the steady-state emission spectra of guanylated fluorophore (A) ($\lambda_{ex}$ 272 nm) at different pH values, as tested in Example 2, and the corresponding plot of the $\lambda_{em}$ for each measured pH (insert).

Figure 5a shows the fluorescence variation of guanylated fluorophore (A) upon addition of 100 $\mu$M ions in 10 $\mu$M as tested in Example 3. Figure 5b shows absorbance spectra of guanylated fluorophore (A) upon successive addition of magnesium sulfate (0 - 100 $\mu$M) in 10 $\mu$M water solution of guanylated fluorophore (A) as tested in Example 3. Figure 5c correlates to fluorescence spectra of guanylated fluorophore (A) upon successive addition of magnesium sulfate (0 - 100 $\mu$M) in 10 $\mu$M aqueous solution of guanylated fluorophore (A) as tested in Example 3. Figure 5d shows the fluorescence intensity of guanylated fluorophore (A) upon gradual addition of sulfate plotted in a typical Stern-Volmer (SV) plot and the modified SV equation plot (insert)($\lambda_{ex}$ 272 nm), as tested in Example 3. The ratio of sulfate concentration to guanylated fluorophore A concentration is indicated as $[SO_4^{2-}]$:[A] ratio in figure 5d.

Figure 6a depicts the fluorescence decrease (%) of guanylated fluorophore (A) (5 $\mu$M) upon addition of 100 $\mu$M anions in water. Figure 6b shows the fluorescence changes (%) of guanylated fluorophore (A) (5 $\mu$M) when successive addition of 100 $\mu$M of anions are added in water. Figure 6c collects the fluorescence increase (%) when real samples are titrated by successive additions of guanylated fluorophore (A): test sample (small graph) and diluted commercial mineral water (big graph) as tested in Example 3.

Figure 7 shows the Scatchard plot of Example 4 for the determination of the association constant for the 1:1 complex of guanylated fluorophore (A) and sulfate anion (i.e., $(A)(SO_4^{2-})$, complex A, Figure 7a), for the a 2:1 complex of guanylated fluorophore (A) and sulfate anion (i.e., $(A)_2(SO_4^{2-})$, complex B, Figure 7b).

**DETAILED DESCRIPTION OF THE INVENTION**

A method as described herein is a method for selectively detecting and, optionally, quantifying sulfate anions in an aqueous sample by fluorescence spectroscopy using a guanylated fluorophore. In particular embodiments, a method as described herein may be a method for selectively quantifying sulfate anions. As a skilled person would understand, quantification of sulfate anions implies their detection.

A fluorophore as used herein may be regarded as a molecular entity that emits fluorescence (i.e., emits radiation in a wavelength when irradiated at a different wavelength).

A guanylated fluorophore is a fluorophore which has been provided with a guanidinyl moiety, by, e.g., subjecting a fluorophore to a guanylation reaction. A guanylated fluorophore may also be obtained by other means.

A guanylated fluorophore suitable for a method as described herein comprises a fluorescent group, said fluorescent group containing a substituted or unsubstituted planar fluorescent core, and a guanidinyl moiety.

A fluorescent group as used herein may be regarded as the part of the guanylated fluorophore responsible for the fluorescent activity of the guanlyated fluorophore. In other words, it may be regarded as the part of the molecule that emits fluorescence. The fluorescent group has a planar fluorescent core which, may be typically formed by a planar conjugated system, more typically comprising rings such as, e.g., a conjugated polycyclic system, in particular, comprising two aromatic rings or three aromatic rings.

In a guanylated fluorophore suitable for a method as described herein a fluorescent group may be typically selected from: a fluorenyl group, a naphthyl group, an anthracenyl group, a naphthalimidyl group, and a coumarinyl group, each being substituted or unsubstituted. Accordingly, the planar fluorescent core of a guanylated fluorphore may respectively be fluorenyl, naphthyl, anthracenyl, naphthalimidyl and coumarinyl, all of which are residues of the corresponding compounds of table 1. Similarly, the guanylated fluorophore may be regarded as a derivative from the corresponding compound of table 1.

Table 1

| fluorene | naphthalene | anthracene |
|---|---|---|
| 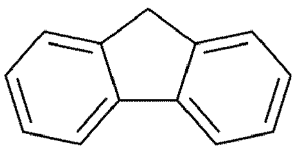 | 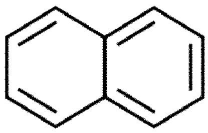 | 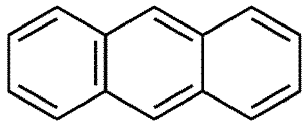 |
| naphthalimide | coumarin | |
| 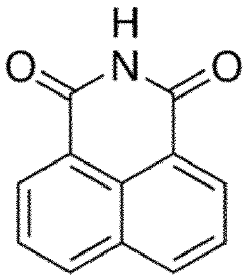 | 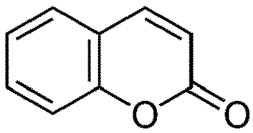 | |

If substituted, the fluorescent group or, in particular, the planar fluorescent core preferably has a substitution selected from an alkyl substitution and an aryl substitution. In particular, the alkyl substitution may be selected from isopropyl, ethyl, tertbutyl, cyclohexyl, and methyl, preferably selected from isopropyl, ethyl and methyl, and/or the aryl substitution may be phenyl. It may be preferred for the planar fluorescent core to be unsubstituted.

In a method as described the fluorescent group of the guanylated fluorophore may be a substituted or unsubstituted fluorenyl group, in particular, may preferably be an unsubstituted fluorenyl group.

A guanidinyl moiety in a guanylated fluorophore suitable for a method as described herein is a residue derived from guanidine which keeps the core structure of guanidine as a guanidinyl group, which may be substituted in one or more positions.

Guanidine structure:

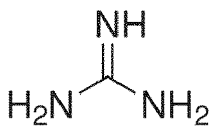

In a guanylated fluorophore suitable for a method as described herein a guanidinyl moiety may be typically selected from an alkyl substituted guanidinyl group, an unsubstituted guanidinyl group, and an aryl substituted guanidinyl

group.

In particular embodiments, a guanidinyl moiety may be selected from an alkyl substituted guanidinyl group and an aryl substituted guanidinyl group.

More in particular, a guanidinyl moiety may be a guanidinyl group having one alkyl substitution or having two alkyl substitutions, and may preferably be a guanidinyl group having two alkyl substitutions.

In several embodiments, the one or two alkyl substitutions may be selected from an isopropyl substitution, an ethyl substitution, a tertbutyl substitution, a cyclohexyl substitution, and a methyl substitution, in particular selected from an isopropyl substitution, an ethyl substitution and a methyl substitution, and preferably is an isopropyl substitution. A guanidinyl moiety having two alkyl substitutions may be a bicyclic guanidinyl group. However, the guanidinyl moiety may preferably have two isopropyl substitutions.

In a method as described herein, a guanylated fluorophore may have the following structure (1):

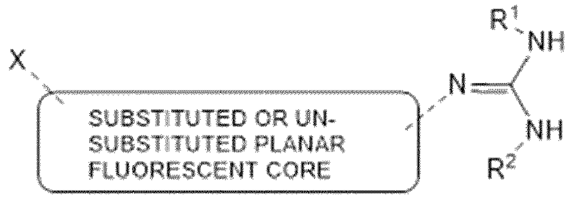

(1)

wherein

$R^1$ and $R^2$ are independently selected from H, alkyl, and aryl

X is selected from H, or a guanidinyl moiety, and

wherein, if substituted, the planar fluorescent core may have a substitution selected from an alkyl substitution and an aryl substitution, and, in particular, the alkyl substitution may be selected from isopropyl, ethyl, tertbutyl, cyclohexyl, and methyl, preferably selected from isopropyl, ethyl and methyl, and/or the aryl substitution may be phenyl.

Thus, if present, the substitutions in the fluorescent core of structure (1) correspond to the substitutions indicated above for the fluorescent group.

In several embodiments, one or both of $R^1$ and $R^2$ of structure (1) are alkyl and may be selected from the specific alkyl substitutions indicated above for the guanidinyl moiety.

A guanylated fluorophore for a method as described herein may comprise one guanidinyl moiety. If the guanylated fluorophore comprises one guanidinyl moiety, then, X in structure (1) is H.

Alternatively, the guanylated fluorophore may comprise more than one guanidinyl moiety, and in particular may comprise two guanidinyl moieties. If the guanylated fluorophore comprises two guanidinyl moieties, then, X in structure (1) is a guanidinyl moiety which may be a substituted or unsubstituted guanidinyl moiety as discussed above. If the guanylated fluorophore comprises more than one guanidinyl moiety, and in particular two guanidinyl moieties, the guanidinyl moieties may be the same or may be different, although they may preferably be the same.

In a particular embodiment, a guanylated fluorophore may comprise a fluorescent group and a guanidinyl moiety, wherein the fluorescent group is a substituted or unsubstituted fluorenyl group and the guanidinyl moiety is selected from an unsubstituted guanidinyl group, an alkyl substituted guanidinyl group and an aryl substituted guanidinyl group. Such guanylated fluorophores have been found to have a high fluorescence quenching in the presence of sulfate anions, in particular a quenching of 40-50 % of the total fluorescence intensity of the guanylated fluorophore for a molar ratio of sulfate to guanylated fluorophore of, e.g., 10:1. Such guanylated fluorophores have also been found to form complexes with sulfates of high stability.

Accordingly, the present invention relates to such a guanylated fluorophore.

A guanylated fluorophore as described herein may particularly have the following structure (2)

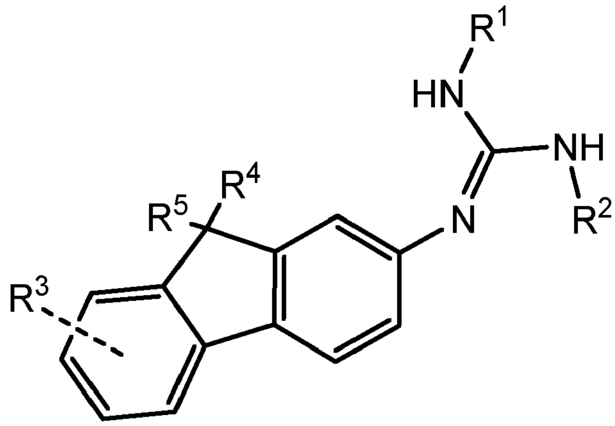

(2)

wherein $R^1$, $R^2$, $R^3$, $R^4$, and $R^5$ are selected from H, alkyl and aryl.

In structure (2), alkyl may preferably be selected from isopropyl, ethyl, and methyl and aryl may preferably be phenyl.

In structure (2), $R^3$, $R^4$ and $R^5$ may preferably be H.

In several embodiments the alkyl substituted guanidinyl group of a guanylated fluorophore as described herein, comprises one alkyl substitution or two alkyl substitutions, and preferably comprises two alkyl substitutions. Accordingly, in a guanylated fluorophore of structure (2) at least one of $R^1$ and $R^2$ may be alkyl or both $R^1$ and $R^2$ may be alkyl.

The alkyl substitutions may be as defined above for a guanylated flurophore of a method as described herein. In several particular embodiments, the one or two alkyl substitutions, such as $R^1$ and/or $R^2$ of structure (2), may be selected from an isopropyl substitution, an ethyl substitution, and a methyl substitution. More in particular an alkyl substituted guanidinyl group, may comprise two isopropyl substitutions. Without being bound to any theory it is believed that the presence of isopropyl substitutions may further contribute to the stability of the complex between sulfate and the guanylated fluorophore. For instance, an affinity constant of $Ka = 1.26x10^5$ has been measured for a 1:1 complex of 2-(9H-fluoren-2-yl)-1,3-diisopropylguanidine (guanylated fluorophore (A)) and sulfate anion (i.e., $(A)(SO_4^{2-})$), and an affinity constant of $Ka = 1.55x10^{10}$ has been measured for a 2:1 complex of guanylated fluorophore (A) and sulfate anion (i.e., $(A)_2(SO_4^{2-})$).

More in particular the guanylated fluorophore as described therein may be of the following structure (3):

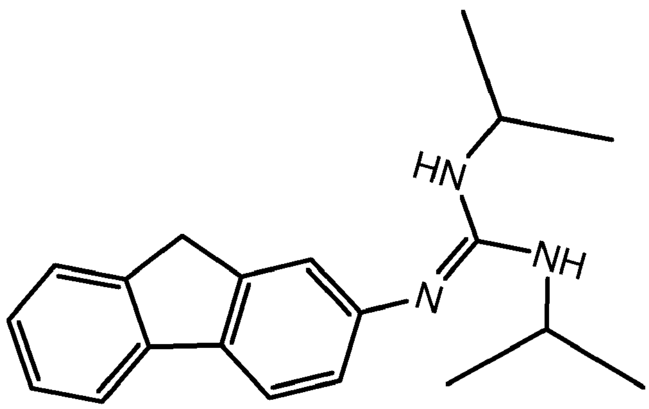

(3)

Guanylated fluorophores with one guanidinyl moiety as described herein, and in particular of structure (3), which chemical name corresponds to 2-(9H-fluoren-2-yl)-1,3-diisopropylguanidine, also referred herein as guanylated fluorophore (A), have been found to have an increased sensitivity and selectivity for sulfate anion detection quantification, in particular, when compared to, e.g., guanylated fluorophores with two guanidinyl moieties.

Typically, the wavelengths of excitation and detection will depend on the type of fluorescent core of the guanylated fluorophore. For instance, if the fluorescent core is derived from fluorene, the excitation weave length may be from 250 to 290 nm, more in particular from 260 to 280 nm, and more in particular of about 272 nm and the detection wavelength may be from 350 to 390 nm, in particular from 360 to 380 nm, more in particular of about 370 nm.

As indicated above and as discussed in more detail below, guanylated fluorophores as described herein are useful for the detection and quantification of sulfate anions. Accordingly, the instant invention also relates to the use of a guanylated fluorophore as defined herein as a sulfate sensor.

A method as described herein for selectively detecting and, optionally, quantifying sulfate anions in an aqueous sample by fluorescence spectroscopy, may be used for any aqueous sample of interest.

For instance, an aqueous sample may be selected from an environmental sample or a sample prepared from the environment (e.g., a sample of a water source, such as a river, or a sample of industrial waste water), a biological sample or prepared from a biological system (e.g., a physiological sample or preparation thereof), a sample of a food product or prepared from a food product (e.g., a juice), a sample of a toiletry product or a sample prepared from a toiletry product, (e.g., a shower gel), a sample of drinking water (e.g. water of drinking water sources, bottled water or tap water).

A method as described herein may be used to detect and, optionally, to quantify sulfate anion in an aqueous sample comprising sulfate anions, wherein the concentration of sulfate anion in the aqueous sample may be from 0.1 to 100 mg/L (i.e., 1 to 1000 $\mu$M), in particular from 0.1 to 10 mg/L (i.e., 1 to 100 $\mu$M), and more in particular from 0.1 to 1 mg/L (i.e., 1 to 10 $\mu$M). Such concentrations are well below the concentrations detected and quantified by known methods such as methods using barium chloride, for instance the gravimetric method using barium chloride is typically used for concentrations above 10 mg/L, the turbidimetric method using barium chloride is used for concentrations above 60 mg/L and the volumetric methods using barium chloride is used for concentrations above 100 mg/L. A skilled person working with aqueous samples containing sulfate anions may typically know in which range lies the sulfate anion concentration of aqueous samples of interest. Nonetheless, a skilled person may also estimate the concentration range of sulfate in a sample of interest (e.g., using other known methods, typically less accurate than a method as described herein) and then quantify the sulfate concentration with a method as described herein.

As a skilled person would understand samples with higher concentrations of sulfate may be diluted in water (to concentrations as indicated above from 1 to 1000 $\mu$M, in particular from 1 to 100 $\mu$M, and more in particular from 1 to 10 $\mu$M) to make the fluorescent measurements in a method as described herein. Dilution can then be taken into account for the quantification of sulfate in the original sample.

A method as described herein has been found to allow the detection and quantification of low concentrations of sulfate, which are significantly lower to concentrations detected and quantified with typical methods for sulfate detection such as those using barium salts, e.g., precipitation with barium salts. Such sensibility for sulfate anion detection and quantification may allow for detection and quantification of sulfate anions when present in low concentrations in a sample or may also allow reducing the amount of sample needed for detection and/or quantification of sulfate anion.

It has also been found that detection and, optionally, quantification of sulfate anions by a method as described herein works independently of the counter ions present in the aqueous sample. Accordingly, a method as described herein may be used for the detection and optionally quantification of sulfate anions in an aqueous sample comprising any cations, e.g., making up the sulfate salts present in the aqueous sample. For instance, the aqueous sample may comprise cations selected from magnesium cations ($Mg^{2+}$), copper cations ($Cu^{2+}$), nickel cations ($Ni^{2+}$), calcium cations ($Ca^{2+}$), sodium cations ($Na^{+}$) and potassium cations ($K^{+}$).

A method as described herein has also been found to be able to detect and optionally quantify sulfate anions selectively. In particular, it has been found that the presence of anions other than sulfate in the aqueous solution, such as chloride ($Cl^{-}$), carbonate ($CO^{3-}$), nitrate ($NO^{3-}$), phosphate ($PO_4^{3-}$), hydrogen phosphate ($HPO_4^{2-}$ and/or $HPO_4^{-}$), thiocyanate ($SCN^{-}$), sulfite ($SO_3^{2-}$), sodium dodecyl sulfate (SDS) (also known as sodium lauryl sulfate (SLS)) and/or acetate ($CH_3COO^{-}$), or mixtures thereof, does not interfere with the detection and quantification of the sulfate anions. Accordingly, a method as described herein may be particularly suitable for detecting and, optionally, quantifying sulfate anions ($SO_4^{2-}$) in an aqueous sample comprising anions other than sulfate, in particular comprising an anion selected from chloride ($Cl^{-}$), carbonate ($CO_3^{-}$), nitrate ($NO_3^{-}$), phosphate ($PO_4^{3-}$), hydrogen phosphate ($HPO_4^{2-}$, or $HPO_4^{-}$), other sulfur containing anions such as thiocyanate ($SCN^{-}$), sulfite ($SO_3^{2-}$) and sodium dodecyl sulfate (SDS), and/or organic acid anions such as acetate ($CH_3COO^{-}$).

A method as described herein has also been found to be able to detect and optionally quantify sulfate anions over a wide pH range. In particular, a guanylated fluorophore as described herein has been found to display suitable fluorescence intensity for sulfate anion detection and quantification for aqueous samples of a pH from 2.5 to 13, in particular of a pH from 4.0 to 12.5, and more in particular of a pH from 5.0 to 12.0. Accordingly, aqueous samples used in a method as described herein may have a pH from 2.5 to 13, in particular of a pH from 4.0 to 12.5, and more in particular of a pH from 5.0 to 12.0.

Fluorescence spectroscopy suitable for a method as described herein may be carried out by any means and by any methods known in the art.

In particular, the inventors have found that guanylated fluorophores as described herein have their fluorescence reduced in the presence of sulfate anions or, in other words, their fluorescence is quenched by sulfate anions. Accordingly, in a method as described herein the presence and optionally the concentration of sulfate anion may be determined by monitoring said quenching by of the guanylated fluorophore may be followed, e.g., by fluorescence spectroscopy.

For instance, a method as described herein may typically comprise adding a guanylated fluorophore to an aqueous sample comprising sulfate anions and monitoring the fluorescence of the resulting sample. Monitoring the fluorescence may be done by measuring the fluorescence of the resulting sample containing both the sulfate anion and the guanylated fluorophore, and comparing the fluorescence of the resulting sample with the fluorescence of a reference aqueous sample. The reference aqueous sample may be the starting aqueous sample prior to addition of the guanylated fluorophore, to monitor the increase of fluorescence upon addition of the guanylated fluorophore. Alternatively, the reference aqueous sample may be an aqueous sample of the same concentration of guanylated fluorophore without any sulfate anion, to monitor the decrease of fluorescence in the presence of sulfate anion. A reduction of fluorescence for the tested aqueous sample compared to the reference sample would be indicative of the presence of sulfate anion. Such increase or reduction of fluorescence may be used for detecting and, optionally, quantifying the presence of sulfate anions in a method as described herein.

In particular, a spectrophotometer, and more in particular a spectrofluorometer may be used for fluorescence measurements.

Steady State fluorescence (SSF) spectra can be obtained by subjecting the aqueous sample constant source of light, excited by a emit fluorescence, and the emitted photons, or intensity, are detected as a function of wavelength. SSF spectra may be recorded to determine the fluorescence spectra of the guanylated fluorophore. Then fluorescence intensity may be measured at a selected wavelength - for instance at the wavelength of the maximum fluorescence intensity - in order to obtain a calibration curve, e.g., using solutions of the guanylated fluorophore and known concentrations of sulfate anion. SSF may also be used to determine an unknown sulfate concentration by titration with a guanylated fluorophore as, e.g., described in detail here below.

It is within the scope of skilled person to perform appropriate fluorescence measurements. As a mode of example, fluorescence spectroscopy, and in particular SSF spectra, may be performed using, e.g., 10 mm quartz cuvette to hold the sample for the recording of the spectra and data in a spectrofluorometer which may be equipped with a microchannel plate-photomultiplier tube (MCP-PMT) detector and a time-correlated single photon counting (TCSPC) data acquisition card. As a light source a Xe lamp may be used. A temperature-controlled cuvette holder may be used to maintain the temperature of the sample during the measurement, to a temperature of, e.g., 20-25 °C which corresponds to 293-298 K. Excitation and emission slits may be typically fixed at, e.g., 1 nm. Other parameters such as the step and dwell time may be of, e.g., 1 nm and 0.1 s, respectively.

As indicated above, the wavelength of excitation and detection will depend on the type of fluorescent core of the guanylated fluorophore. For instance, if the fluorescent core is derived from fluorene, the excitation weave length may particularly be of about 272 nm and the detection wavelength may be of about 370 nm.

A method as described herein may comprise titrating the aqueous sample with the guanylated fluorophore. In particular, titration may be used for the quantification of sulfate anions.

Titration may be performed by methods known in the art using lab-scale or industrial scale set-ups. In particular, as a mode of example, titrating may be performed by successively adding to from 1 to 5 mL, in particular 3 mL of an aqueous sample comprising sulfate anions a plurality of equal volumes (e.g., from 1 to 5 μL, in particular 2 to 4 μL, more in particular 3 μL) of a solution of guanylated fluorophore of a known concentration (e.g., 0.1 to 5 mM, 0.5 to 2 mM, more in particular of 1 mM), gradually increasing the concentration of the guanylated fluorophore in the test sample from 0 to a concentration sufficient to establish a full titration curve (e.g., of at most 50 μM, in particular at most 25 μM, more in particular at most 15 μM). The fluorescence spectrum of the resulting solution after each addition may be measured with an excitation wavelength appropriate for the fluorescent core of the guanylated fluorophore (e.g., of 272 nm for fluorene), by detecting the emitted fluorescence in the range of 300 to 500 nm. For each spectrum the value of fluorescence (F) at its maximum (e.g., at 370 nm for fluorene) may be taken, divided by the fluorescence value in absence of the guanylated fluorophore ($F_0$) and multiplied by 100 to obtain the % of fluorescence increase in the presence of the guanylated fluorophore ($(F/F_0)*100$). The % fluorescence increase may be plotted against the corresponding concentration of the guanylated fluorophore of the resulting solution after each addition of guanylated fluorophore to the sulfate containing aqueous sample (see figure 6c as a mode of example). The plotted curve then shows an inflection point which is the intersection point defined by the two straight lines before and after said intersection point. The inflection point corresponds to the point wherein the concentration ratio of guanylated fluorophore to sulfate is 1, and therefore provides the concentration value of the sulfate concentration of the measured sample (e.g., 7.42 μM for the big graph in figure 6c and 5.01 μM for the small graph in figure 6c).

It has been found that such titration methods provide a very good determination of the sulfate concentration in aqueous samples, present in concentrations, e.g., well below 10 mg/L (i.e., below 10 μM). Accordingly, such titration methods are particularly suited for quantification of sulfate anions in an aqueous sample, and particularly useful in the

quantification of sulfate anions in aqueous samples with a low sulfate anion concentration (e.g., from 1 to 10 μM).

Guanylated fluorophores for use in a method described herein may be prepared by known methods.

Nonetheless, the instant invention further relates to a method for preparing a guanylated fluorophore as described herein, in particular a guanylated fluorophore comprising a fluorescent group and a guanidinyl moiety, wherein the fluorescent group is a fluorenyl group and the guanidinyl moiety is selected from an unsubstituted guanidinyl group, an alkyl substituted guanidinyl group and an aryl substituted guanidinyl group.

A method of preparation as described herein may comprise: guanylating a mono-amine containing a fluorene group, by reacting the mono-amine containing the fluorene group with *N,N'*-diisopropyl-carbodiimide, in the presence of a catalyst, in particular, a catalyst selected from $ZnEt_2$, $^nBuLi$, $MgBu_2$, trispentafluorophenylborane, and $Zn(OTf_2)$. The catalyst may preferably be $ZnEt_2$ because of its high activity and selectivity.

The mono-amine containing a fluorene group may preferably be 2-amino fluorene because this precursor has been found to present appropriate characteristics to act as a fluorescent group and can be easily transformed into a guanidine derivative, following the described methodology.

Reacting the mono-amine containing the fluorene group with *N,N'*-diisopropylcarbodiimide, in the presence of a catalyst, may be performed by means known in the art.

As a mode of example, the reaction may be typically performed in a solvent, such as THF, toluene or benzene, the solvent may preferably be THF because of its polarity. The reaction may be performed in the presence of a catalyst (as detailed above) in a reactor, such as a Schlenk tube, under protective atmosphere (e.g., of dry nitrogen or argon). As a mode of example, the reaction may be performed at a temperature from 20 to 80 °C, in particular from 30 to 70 °C, and more in particular from 40 to 60 °C. The reaction may be monitored by NMR analysis of aliquots of the reaction mixture and may be stopped when the signals of the reagents disappear in the NMR spectra of these aliquots. The reaction time may be from 0.5 to 6 h, in particular from 1 to 5 h, more in particular from 2 to 4 h. For instance, the reaction may be stopped by stopping any heating, concentrating the reaction mixture and adding a solvent that facilitates precipitation and/or crystallization of the guanylated fluorophore product such as hexane. Crystallisation may be favoured by keeping the mixture obtained at low temperature, e.g., at a temperature from -50 to 5 °C, in particular from -40 to -10 °C. The guanylated fluorophore may be obtained yields from 80 to 99% in particular, from 85 to 98 % and more in particular from 90 to 95 %.

## EXAMPLES

In the following, the invention will be further illustrated by means of examples. The examples should in no case be interpreted as limiting the scope of the invention, but only as an illustration of the invention.

### General procedures.

Synthesis reactions were performed using standard Schlenk and glove-box techniques under an atmosphere of dry nitrogen. Tetrahydrofuran and hexane were pre-dried over sodium wire and distilled under nitrogen from sodium-potassium alloy (hexane), and sodium-benzophenone (THF). $CDCl_3$ was stored over activated 4 Å molecular sieves and degassed by several freeze-thaw cycles.

For experiments in water, stock solutions (1 mM) of guanylated fluorophore (A) in ethanol were used. All aqueous solutions were prepared by diluting the stock solution in Milli-Q water to the desired concentration and filtered with 0.22 μm filters before use. For the pH titration experiments, aqueous solutions of guanylated fluorophore (A) (10 μM) were titrated by the successive addition of small volumes (in the order of microliters) of HCl and NaOH solutions of different concentrations (0.01-10 M) to an initial volume of 25 mL to minimize changes in the sample volume. UV-Vis absorption, SSF spectra were recorded.

For ion interaction experiments, an aqueous solution of guanylated fluorophore (A) (10 μM) was titrated with different salt solutions ($CaCl_2$, KCl, NaCl, $NaCH_3COO$, KSCN, $Na_2CO_3$, $Na_2SO_3$, SDS, $Na_2HPO_4$, $Na_3PO_4$, $AgNO_3$, $MgSO_4$, $CuSO_4$, and $NiSO_4$). Different concentrations of ions were tested from 0 to 100 μM and steady-state fluorescence (SSF) spectra were acquired. All measurements were performed using 10 mm quartz cuvette (Hellma Analytics).

For the sulfate anion quantification of real samples, a 5μM test sample aqueous solution of sulfate was prepared from commercial $MgSO_4$ and titrated (n = 3) by the successive addition of small volumes (in the order of microliters) of stock solution of guanylated fluorophore (A) of 1 mM in the quartz cuvette. A similar procedure was carried out for the titration of commercial mineral water samples. These samples of 5.5 and 7.5 μM sulfate anion concentration were prepared by dilutions of commercial mineral water, with labelling sulfate values of 1.20 mM (115 mg/L), in milli-Q water.

All NMR experiments were conducted in deuterated solvents at 297 K (which corresponds to 23.85 °C) in a Varian FT 400 spectrometer equipped with a 5-mm triple-nucleus (TXI) probe head with z-gradient coil with a maximum gradient strength of 50 $G/cm^{-1}$. The 1H $\pi/2$ pulse length was adjusted for each sample.

Mass spectroscopic analyses were performed on an Advion expression CMS instrument (electron impact).

IR experiments were conducted on a FT/IR 4000 Series Jasco Instruments.

UV-Vis absorption spectra of guanylated fluorophore (A) were recorded at room temperature using a Cary 100 (Varian) spectrophotometer using a slit width of 0.4 nm and a scan rate of 600 nm/min.

Steady-state fluorescence (SSF) spectra of the samples were recorded employing an FLS920 (Edinburgh Instruments) spectrofluorometer equipped with a MCP-PMT (microchannel plate-photomultiplier tube) detector (R3809 model) and a TCSPC (time-correlated single photon counting) data acquisition card (TCC900 model). A TLC 50 temperature-controlled cuvette holder (Quantum Northwest) was employed for the measurements (temperature was controlled at 296 K, which corresponds to 22.85 °C). A Xe lamp of 450 W was used as the light source for SSF spectra. The excitation and emission slits were both fixed at 1 nm, the step and dwell time were 1 nm and 0.1 s, respectively.

**Example 1: Synthesis and Characterization of 2-(9H-fluoren-2-yl)-1,3-diisopropylguanidine (guanylated fluorophore (A))**

0.04 mL of a solution of $ZnEt_2$ in hexane (1M) was added to a solution of 2-aminofluorene (2 mmol) in dry THF (20 mL) in a Schlenk tube. N,N'-diisopropylcarbodiimide (2 mmol) was then added to the above reaction mixture. The reaction was carried out at 50 °C for 3 h. The solution was concentrated under reduced pressure, hexane was added, and the mixture was placed in a refrigerator at -30°C for 16 h. After filtration, the guanylated fluorophore products were obtained as white microcrystalline solids in 98% yield.

1H NMR (400MHz, $CDCl_3$, 297K), $\delta$: 7.68 (m, 2H, H7, H4), 7.49 (d, 3JHH = 7.4 Hz, 1H, H10), 7.33 (t, 3JHH = 7.5 Hz, 1H, H8), 7.21 (t, 3JHH = 7.4 Hz, 1H, H9), 7.05 (s, 1H, H1), 6.87 (d, 3JHH = 8.0 Hz, 1H, H3), 3.84 (s, 2H, H12), 3.81 (bs, 2H, CH(CH3)2), 3.61 (bs, 2H, NH), 1.18 (d, 3JHH = 6.3 Hz, 12H, CH(CH3)2). 13C NMR (126 MHz, C6D6, 297K) $\delta$: 150.53 (C14), 149.38, 144.84, 142.73, 142.45, 137.13, 135.20, 126.56, 125.20, 124.72, 121.88, 119.99, 118.88 (C1-C11, C13), 42.95 (C15), 36.61 (C12), 22.91 (C16, C17). M. p. 257-258°C. HRMS ($ESI^+$): calcd. m/z 308.2127 $[M^+H]^+$; found: 308.2163. IR Neat: $v_{max}$ 3356 (N-H), 1617 (C=N), 1058 (C-N), 1452, 1256, 1151, 869, 727 $cm^{-1}$.

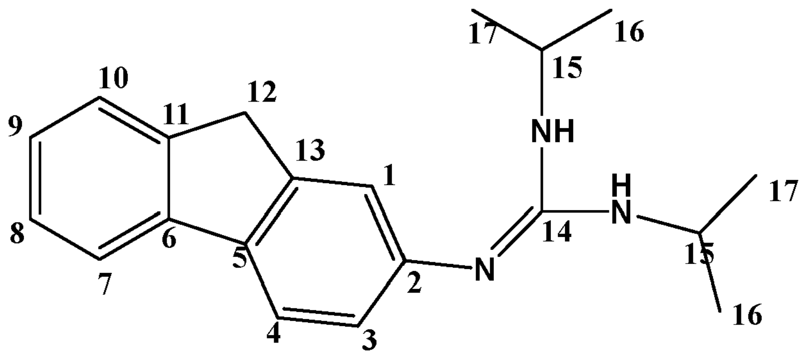

1H NMR and 13C NMR spectra of the product obtained are shown in Figure 1 and Figure 2 respectively.
IR Neat spectrum of the product obtained is shown in Figure 3.

**Example 2: Photophysical properties: pH dependence.**

$AH^+$ will be noted as the protonated form of guanylated fluorophore (A). Figure 4a shows the absorption spectra of guanylated fluorophore (A) at wide broad range of pH. guanylated fluorophore (A) presented a lowest energy band located at 272 nm with a shoulder at 300 nm, related to typical $\pi \rightarrow \pi^*$ electronic transitions in the fluorene core in water at pH 7.36. When the pH is gradually increased, both absorption bands at ~ 270-280 nm decreased and red-shifted on the spectra occur due to an enhancement of red shoulder intensity. Furthermore, an isosbestic point at ~ 280 nm is found for guanylated fluorophore (A). The existence of this isosbestic point in absorbance spectra of A along with the changes observed in the relative absorption intensities at different pH, pointed out the presence of two distinct species in equilibrium (A and $AH^+$). The $pK_a$ values obtained for guanylated fluorophore (A) as 11.1. A presented a single emission band located at 370 nm at low pH values which was assigned to $AH^+$. The band intensity increased with pH and red-shifted by 3 nm at pH above pKa.

Figure 4b shows the steady-state fluorescence emission spectra of guanylated fluorophore (A) ($\lambda_{ex}$ 272 nm) at different pH values, for the samples obtained in the same manner as described above for the samples used to obtain the absorbance spectra of figure 4a. The insert of Figure 4b, shows the corresponding plot of the $\lambda_{em}$ (the wavelength of emission) for each measured pH. It can be clearly seen that both the intensity of the fluorescence and the wavelength of emission are only mildly affected by the pH in the range of 5-12.

**Example 3: Guanylated fluorophore (A) as a fluorescent receptor for sulfate anion.**

Ion recognition experiments were carried out by monitoring the absorbance and fluorescence changes of 10 μM aqueous solution of guanylated fluorophore (A) by the addition of 100 μM aqueous solution of several salts (Figure 5). Although the fluorescence intensity of guanylated fluorophore (A) was the same for most of the ions, the presence of sulfate anions (added to a concentration of 100 μM) caused -2-fold fluorescence quenching in guanylated fluorophore (A) (at a concentration of 10 μM) (Figure 5a). In more detail, Figure 5b shows the titration of 10 μM solution in water of guanylated fluorophore (A) using magnesium, copper, and nickel sulfate within 0.2 - 100 μM where no changes were reported in the absorbance spectra (5b). More importantly, for the same titration a gradual fluorescence decrease without any shift in the emission spectra was observed (see Figure 5c). The results highlight two main facts: on one hand the quenching is exclusively produced by the presence of sulfate anions as the fluorescence signal decreases in the same manner for the three cations used, and on the other hand the absence of any changes in the absorption spectra suggest that there is a strong interaction between sulfate and guanylated fluorophore (A) in the excited state (A*) mostly caused by the formation of supramolecular H-bonding anion:receptor complex.

The fluorescence intensity upon gradual addition of sulfate ion was plotted in a typical Stern-Volmer plot (Figure 5d) and a modified Stern-Volmer equation plot (insert in figure 5d). A substantial decrease of fluorescence signal is observed after the addition of sulfate anions.

The selectivity of guanylated fluorophore (A) was tested with different anions (Figure 6a). Firstly, for similar anion geometries, like $HPO_4^{2-}$ or $PO_4^{3-}$ no sensitivity was found. Besides this, for other sulfur anions such $SO_3^{2-}$ or dodecyl sulfate (SDS), sensitivity was not observed. It is important to emphasize that the selectivity of guanylated fluorophore (A) was tested in water, where the presence of cations like $Ca^{2+}$, $Ni^{2+}$, $K^+$, $Na^+$ or $Mg^{2+}$ or anions like $HPO_4^{2-}$ $NO^{3-}$ and $Cl^-$ normally presented in this media will not affect the final recognition values. To evaluate the selectivity to sulfate anions, additional experiments were conducted on water complex samples containing high concentrations of such ions as interfering.

Figure 6b collects the fluorescence changes (%) of guanylated fluorophore (A) when successive quantities of 100 μM of anions are added over 5μM solution of A in water. Figure 6b clearly proves that the emission intensity exclusively decreases when sulfate anions are added, and those values are not related with the type or the amount of other ions are present in the aqueous sample.

Guanylated fluorophore (A) was tested as a sulfate sensor in water samples, with three real sulfate-containing aqueous samples: a) test sample with 5 μM sulfate anion (prepared in the lab from commercial $MgSO_4$), b) two different commercial mineral water samples (5.5 and 7.5 μM respectively) prepared by dilutions in milli-Q water.

Figure 6c shows the fluorescence changes in the titration experiments of both sulfate samples by the successive addition of guanylated fluorophore (A).

Titration was performed by successively adding to 3 mL of an aqueous sample comprising sulfate anions a plurality of equal volumes (3 μL) of a solution of guanylated fluorophore of a known concentration (of 1 mM), gradually increasing the concentration of the guanylated fluorophore in the test sample from 0 to 12 μM. The fluorescence spectrum of the resulting solution after each addition was measured with an excitation wavelength of 272 nm in the range of 300 to 500 nm. For each spectrum the value of fluorescence (F) at its maximum (at 370 nm) was taken. Each F value was divided by the fluorescence value in absence of the guanylated fluorophore ($F_0$) and multiplied by 100 to obtain the % of fluorescence increase in the presence of the guanylated fluorophore (($F/F_0$)*100). The % fluorescence increase was plotted against the corresponding concentration of the guanylated fluorophore of the resulting solution after each addition of guanylated fluorophore to the sulfate containing aqueous sample (figure 6c). The plotted curve showed an inflection point which is the intersection point defined by the two straight lines before and after said intersection point. The inflection point corresponds to the point wherein the concentration ratio of guanylated fluorophore to sulfate is 1, and therefore the concentration value of the sulfate concentration of the measured sample could be determined.

The value obtained for the prepared test sample were 5.07 ± 0.2 μM, whereas, for the commercial mineral water the sulfate concentrations were 5.61 ± 0.1 and 7.42 ± 0.2, respectively, that correspond to an overall sulfate concentration in the commercial bottle of 1.26 ± 0.5 mM in an excellent agreement with the labeling value of 1.20 mM.

The obtained results, displayed in Table 2, match perfectly with the real values highlighting the applicability of guanylated fluorophore (A)as a sulfate sensor in complex water samples.

Table 2

| Sample | Real value | Found value | RDS, % |
|---|---|---|---|
| Test sample | 5 μM | 5.07 ± 0.2 μM | 1.4 |
| Commercial mineral water sample | 1.20 mM (115 mg/L) | 1.26 ± 0.5 mM (121 mg/L) | 5.0 |
| LOD[a] | 0.10 μM | | |

(continued)

| Sample | Real value | Found value | RDS, % |
|---|---|---|---|
| LOQ[b] | 0.34 μM | | |

[a] Limit of detection (LOD) corresponds with three times the standard deviation of the blank.
[b] Limit of quantitation (LOQ) corresponds with ten times the standard deviation of the blank.

**Example 4: Association constant of complexes of guanylated fluorophore (A) and sulfate anion**

The association constant (Ka) was calculated based on the fluorescence titration data at the same emission wavelength of figure 5d of example 3 above, according to the reported Scatchard plot method (Zhou, H.; Zhao, Y.; Gao, G.; Li, S.; Lan, J.; You, J. Highly Selective Fluorescent Recognition of Sulfate in Water by Two Rigid Tetrakisimidazolium Macrocycles with Peripheral Chains. J. Am. Chem. Soc. 2013, 135 (40), 14908-14911. https://doi.org/10.1021/ja406638b and Yang, W.; Yan, J.; Fang, H.; Wang, B. The First Fluorescent Sensor for D-Glucarate Based on the Cooperative Action of Boronic Acid and Guanidinium Groups. Chem. Commun. (Cambridge, United Kingdom) 2003, 2 (6), 792-793. https://doi.org/10.1039/b300098b)by the following equation (i):

$$[SO_4^{2-}] = \frac{1}{nK_a}\frac{1}{[F]_0^{n-1}}\frac{1-\alpha}{\alpha^n}$$

(i)

where; $\alpha$ is defined as the ratio between concentration of free fluorophore (e.g. [F] and initial $[F]_0$ fluorophore concentration, e.g. for the guanylated fluorophore (A), and can be determined from the fluorescence difference at any titration stage; n is the stoichiometry to the studied complex $F_n(SO_4^{2-})_m$.

The data of figure 5d of example 3 was fitted to equation (i) as shown in:

- Figure 7a for the 1:1 complex of guanylated fluorophore (A) and sulfate anion (i.e., $(A)(SO_4^{2-})$, complex A)
- Figure 7b for the a 2:1 complex of guanylated fluorophore (A) and sulfate anion (i.e., $(A)_2(SO_4^{2-})$, complex B)

The association constants found for the complexes of guanylated fluorophore (A) and sulfate are summarized in table 3.

Table 3

| Complex | Structure | Stoichiometry (n:m) | Ka |
|---|---|---|---|
| A | $(A)(SO_4^{2-})$ | 1:1 | $1.26 \times 10^5$ |
| B | $(A)_2(SO_4^{2-})$ | 2:1 | $1.55 \times 10^{10}$ |

## Claims

1. A method for selectively detecting and, optionally, quantifying sulfate anions in an aqueous sample comprising the same by fluorescence spectroscopy using a guanylated fluorophore comprising a fluorescent group containing a substituted or an unsubstituted planar fluorescent core, and a guanidinyl moiety, wherein

   - the fluorescent group is selected from: a fluorenyl group, a naphthyl group, an anthracenyl group, a naphthalimidyl group, and a coumarinyl group, each being substituted or unsubstituted; and
   - the guanidinyl moiety is selected from an alkyl substituted guanidinyl group, an unsubstituted guanidinyl group, and an aryl substituted guanidinyl group

2. The guanylated fluorophore of claim 1 having the following structure (1):

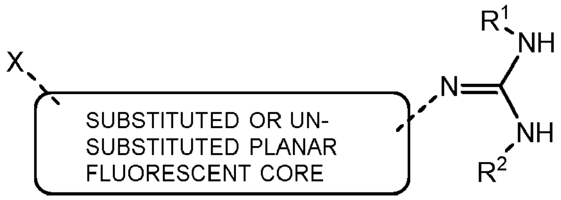

(1)

wherein
$R^1$ and $R^2$ are independently selected from H, alkyl, and aryl
X is selected from H, or a guanidinyl moiety, and
wherein, if substituted, the planar fluorescent core has a substitution selected from an alkyl substitution and an aryl substitution, and, in particular, the alkyl substitution is selected from isopropyl, ethyl, tertbutyl, cyclohexyl and methyl, preferably selected from isopropyl, ethyl, and methyl, and/or the aryl substitution is phenyl.

**3.** The method of claim 1 or 2 wherein the guanylated fluorophore comprises one guanidinyl moiety or the guanylated fluorophore comprises more than one guanidinyl moiety, and in particular comprises two guanidinyl moieties.

**4.** The method of any one of claims 1-3 wherein the fluorescent group of the guanylated fluorophore is a substituted or unsubstituted fluorenyl group, in particular an unsubstituted fluorenyl group.

**5.** The method of any one of claims 1-4, wherein the guanidinyl moiety is selected from an alkyl substituted guanidinyl group and an aryl substituted guanidinyl group, in particular a guanidinyl group having one alkyl substitution or having two alkyl substitutions, preferably a guanidinyl group having two alkyl substitutions and, optionally, wherein the one or two alkyl substitutions are selected from an isopropyl substitution, an ethyl substitution, a tertbutyl substitution, a cyclohexyl substitution, and a methyl substitution, in particular selected from an isopropyl substitution, an ethyl substitution, and a methyl substitution, and preferably is an isopropyl substitution.

**6.** The method of any one of claims 1-5 comprising titrating the aqueous sample with the guanylated fluorophore.

**7.** The method of any one of claims 1-6 wherein the aqueous sample has a sulfate anion concentration from 1 to 1000 $\mu$M, in particular from 1 to 100 $\mu$M, and more in particular from 1 $\mu$M to 10 $\mu$M.

**8.** The method of any one of claims 1-7 wherein the aqueous sample comprises an anion other than a sulfate anion, in particular an anion selected from chloride ($Cl^-$), carbonate ($CO^{3-}$), nitrate ($NO^{3-}$), phosphate ($PO_4^{3-}$), hydrogen phosphate ($HPO_4^{2-}$ and/or $HPO_4^-$), thiocyanate ($SCN^-$), sulfite ($SO_3^{2-}$), sodium dodecyl sulfate (SDS), acetate ($CH_3COO^-$), and mixtures thereof.

**9.** The method of any one of claims 1-8 wherein the aqueous sample has a pH from 2.5 to 13, in particular from 4.0 to 12.5, and more in particular from 5.0 to 12.0.

**10.** A guanylated fluorophore comprising a fluorescent group and a guanidinyl moiety, wherein the fluorescent group is a substituted or unsubstituted fluorenyl group and the guanidinyl moiety is selected from an unsubstituted guanidinyl group, an alkyl substituted guanidinyl group and an aryl substituted guanidinyl group.

**11.** The guanylated fluorophore of claim 10 having the following structure (2)

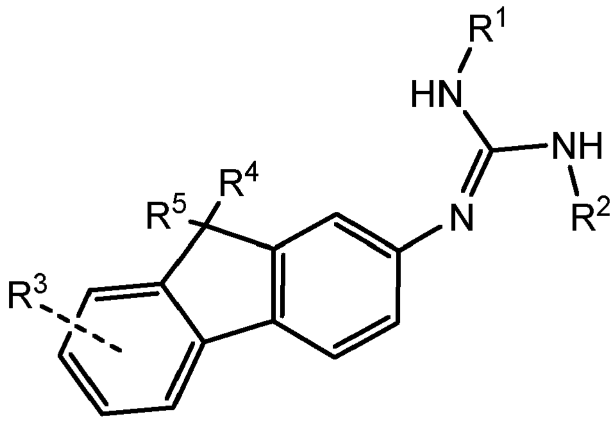

(2)

wherein $R^1$, $R^2$, $R^3$, $R^4$, and $R^5$ are selected from H, alkyl and aryl.

**12.** The guanylated fluorophore of claim 10 or claim 11 wherein the alkyl substituted guanidinyl group, comprises one alkyl substitution or two alkyl substitutions, and preferably comprises two alkyl substitutions.

**13.** The guanylated fluorophore of claim 12 wherein, the one or two alkyl substitutions are selected from an isopropyl substitution, an ethyl substitution, and a methyl substitution, in particular the alkyl substituted guanidinyl group, comprises two isopropyl substitutions and more in particular the guanylated fluorophore is of the following structure (3):

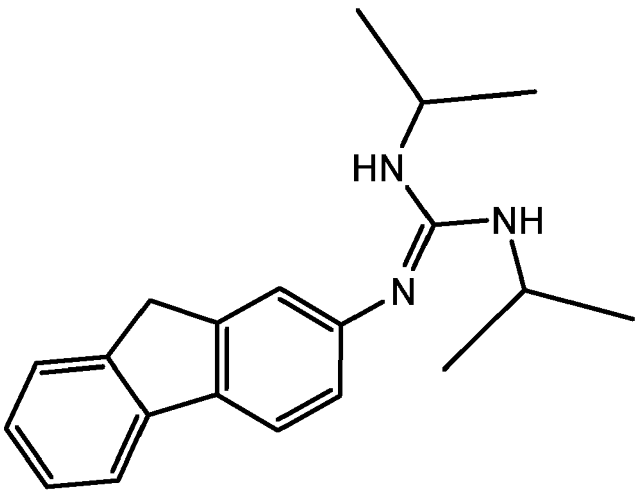

(3)

**14.** A method for preparing a guanylated fluorophore of any one of claims 10-13, comprising: guanylating a mono-amine containing a fluorene group, by reacting the mono-amine containing the fluorene group with *N,N'*-diisopropyl-carbodiimide, in the presence of a catalyst, in particular the catalyst is selected from$ZnEt_2$, $^nBuLi$, $MgBu_2$, trispentafluorophenylborane, and $Zn(OTf_2)$, and more in particular the catalyst is $ZnEt_2$.

**15.** Use of a guanylated fluorophore as defined for the method of any one of claims 1-9 or as defined in any one of claims 10-14, as a sulfate sensor.

FIGURE 1

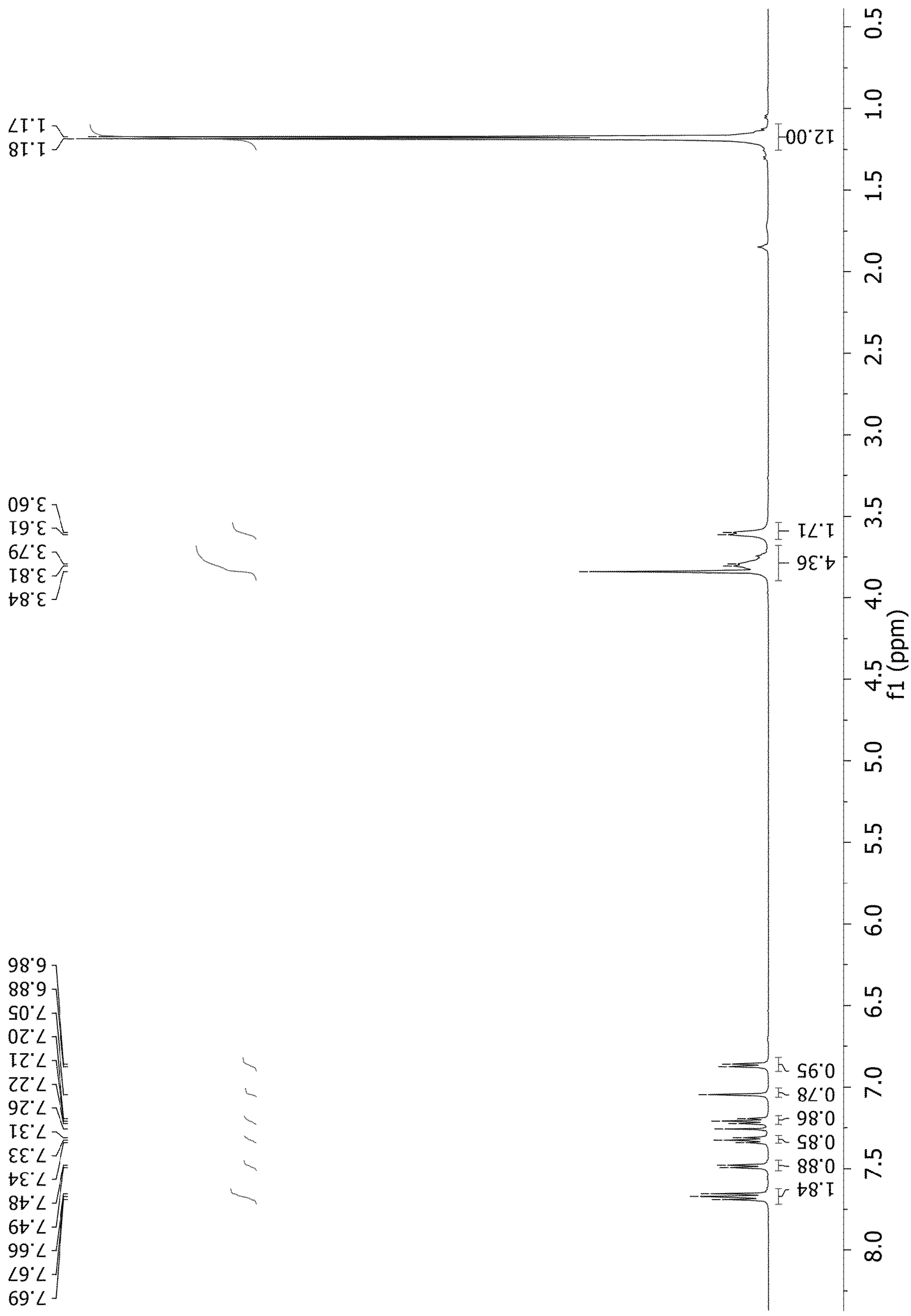

1.17
1.18
3.60
3.61
3.79
3.81
3.84
6.86
6.88
7.05
7.20
7.21
7.22
7.26
7.31
7.33
7.34
7.48
7.49
7.66
7.67
7.69
12.00
1.71
4.36
0.95
0.78
0.86
0.85
0.88
1.84
0.5
1.0
1.5
2.0
2.5
3.0
3.5
4.0
4.5
5.0
5.5
6.0
6.5
7.0
7.5
8.0
f1 (ppm)

FIGURE 2

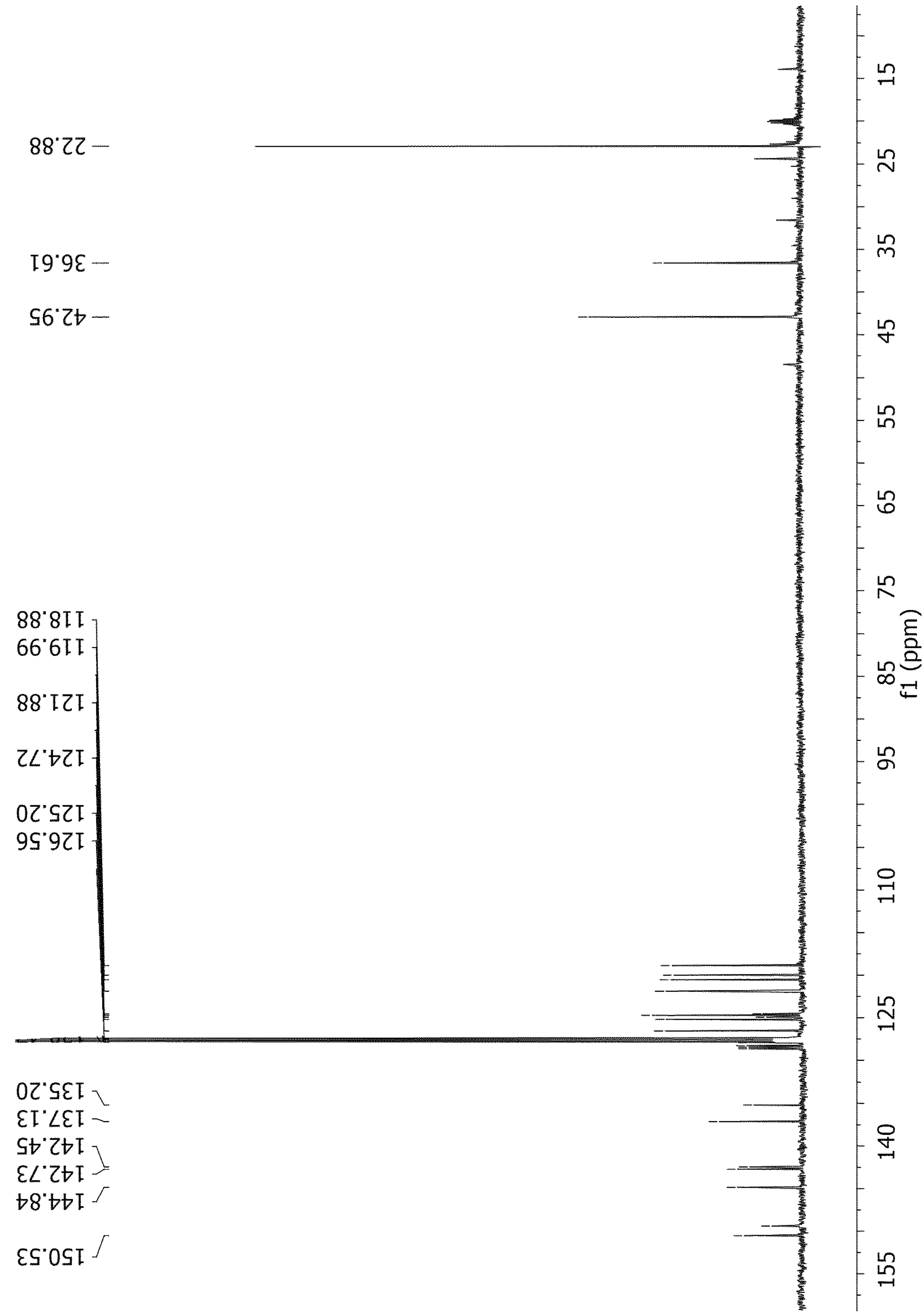
22.88
36.61
42.95
118.88
119.99
121.88
124.72
125.20
126.56
135.20
137.13
142.45
142.73
144.84
150.53
15
25
35
45
55
65
75
85
95
110
125
140
155
f1 (ppm)

FIGURE 3

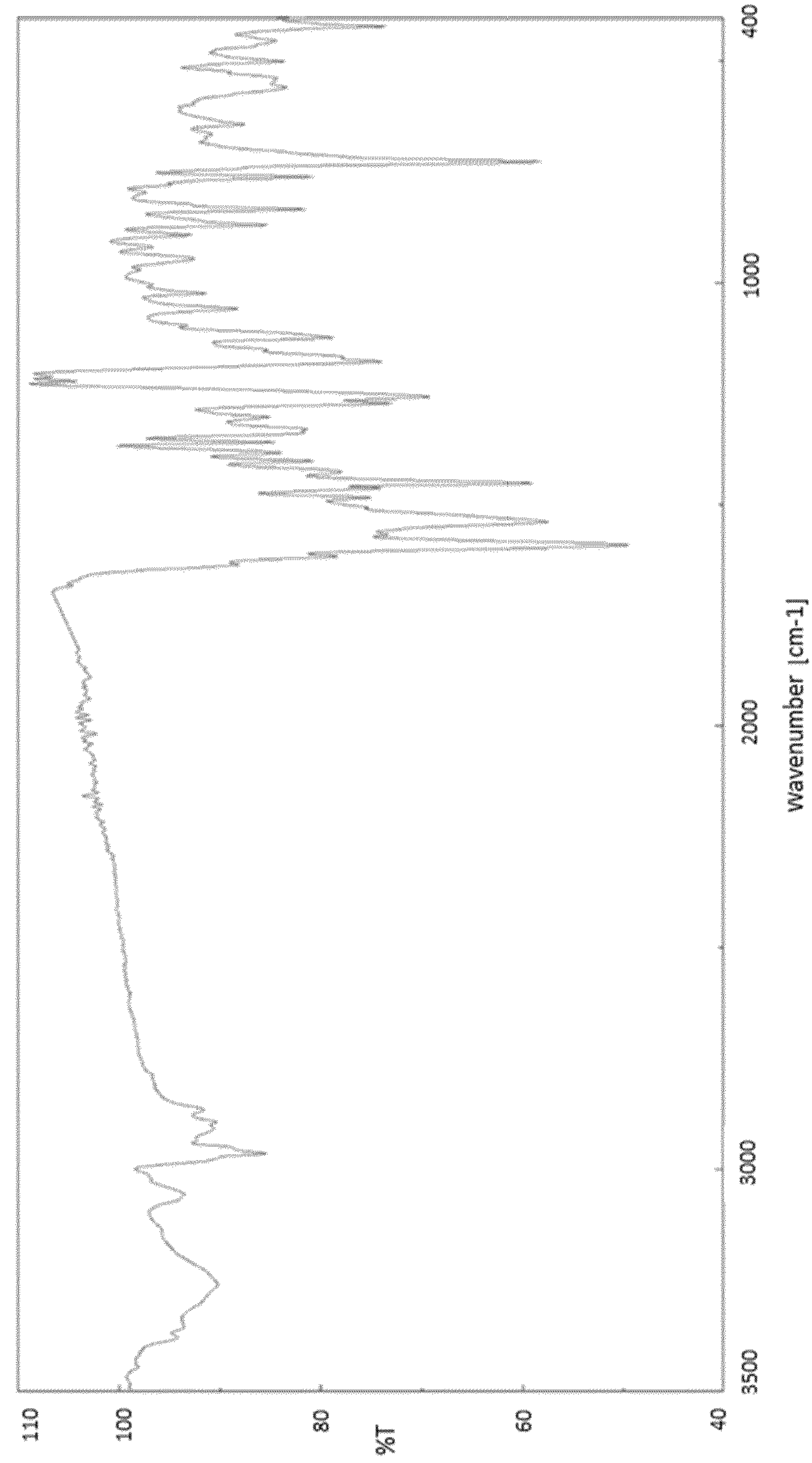

FIGURE 4

a)

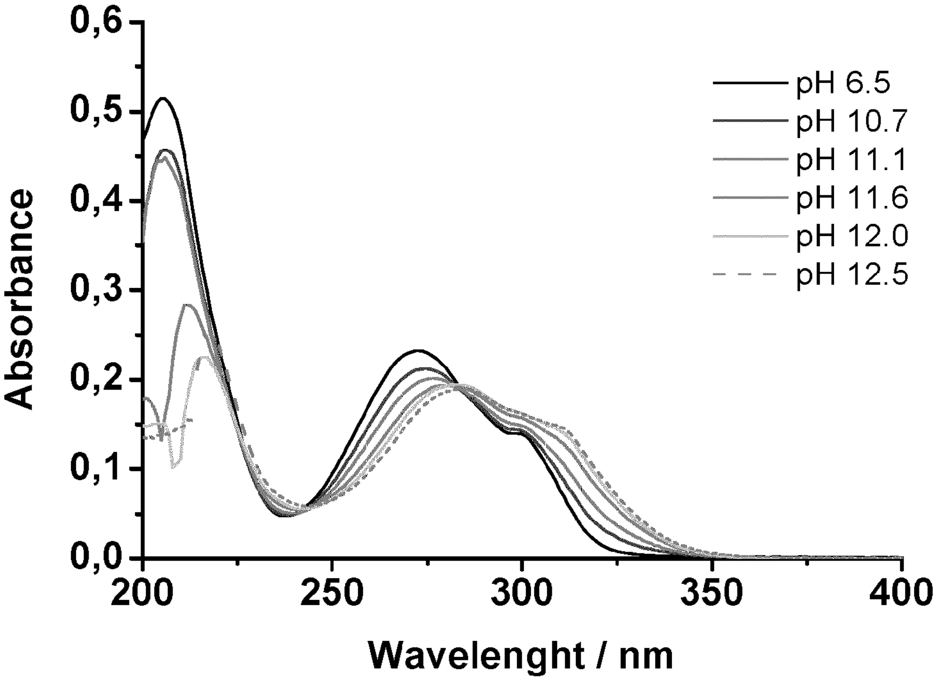
0,6
0,5
0,4
0,3
0,2
0,1
0,0
Absorbance
200
250
300
350
400
Wavelenght / nm
pH 6.5
pH 10.7
pH 11.1
pH 11.6
pH 12.0
pH 12.5

b)

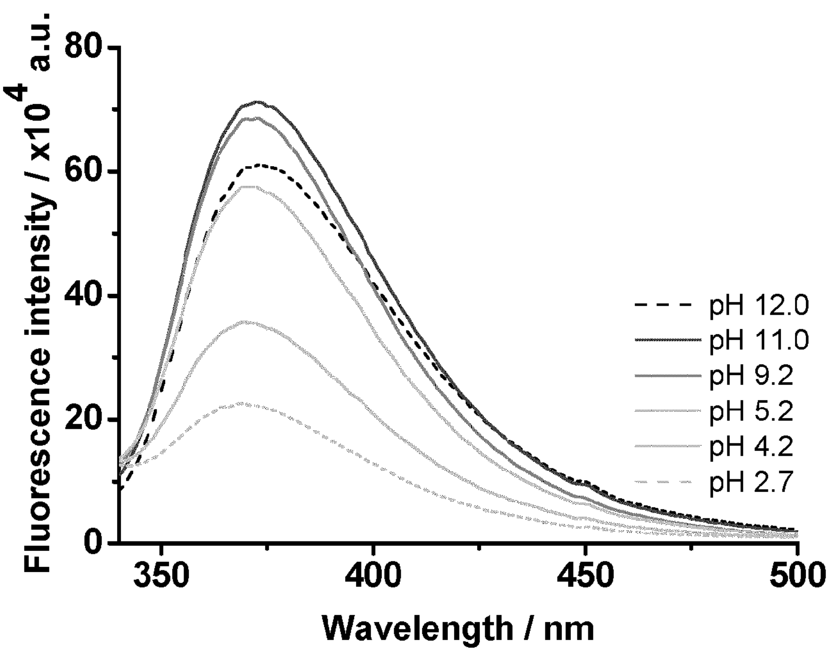
80
60
40
20
0
Fluorescence intensity / x10$^4$ a.u.
350
400
450
500
Wavelength / nm
pH 12.0
pH 11.0
pH 9.2
pH 5.2
pH 4.2
pH 2.7

c)

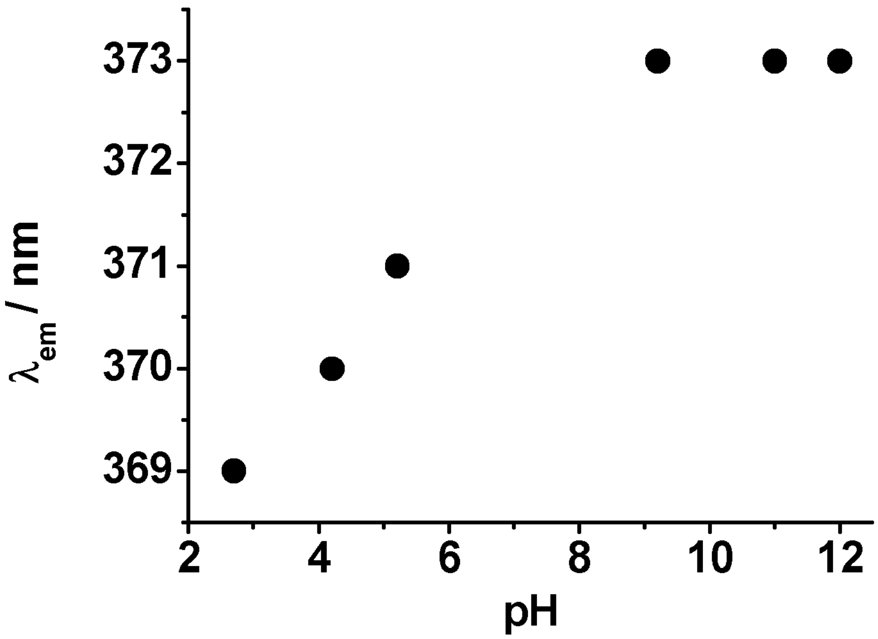

FIGURE 5

a)

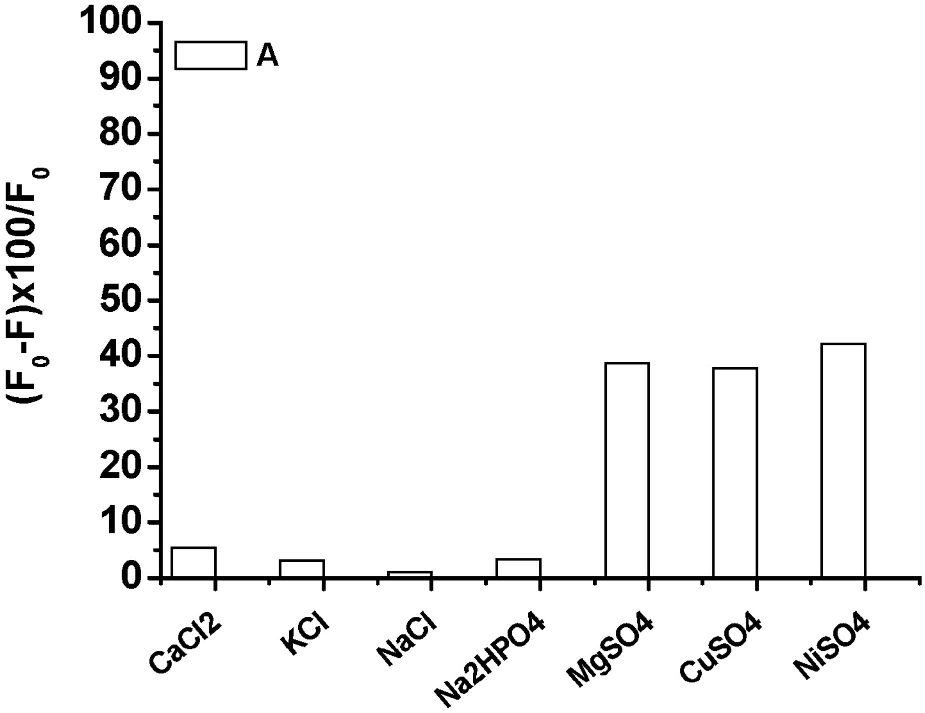
100
90
80
70
60
50
40
30
20
10
0
A
$(F_0-F)x100/F_0$
CaCl2
KCl
NaCl
Na2HPO4
MgSO4
CuSO4
NiSO4

b)

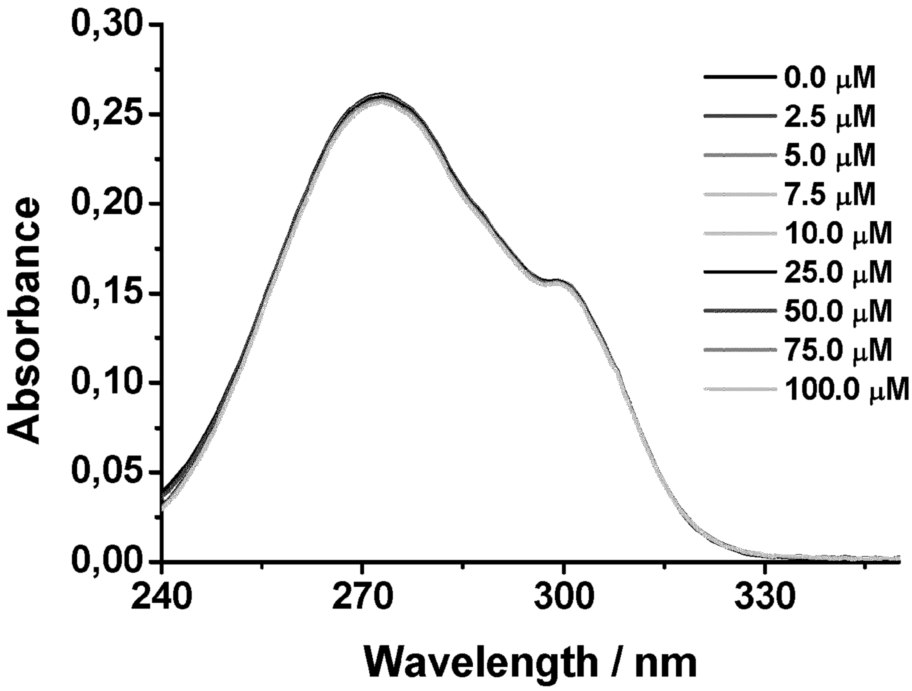
0,30
0,25
0,20
0,15
0,10
0,05
0,00
Absorbance
0.0 μM
2.5 μM
5.0 μM
7.5 μM
10.0 μM
25.0 μM
50.0 μM
75.0 μM
100.0 μM
240
270
300
330
Wavelength / nm

FIGURE 5

c)

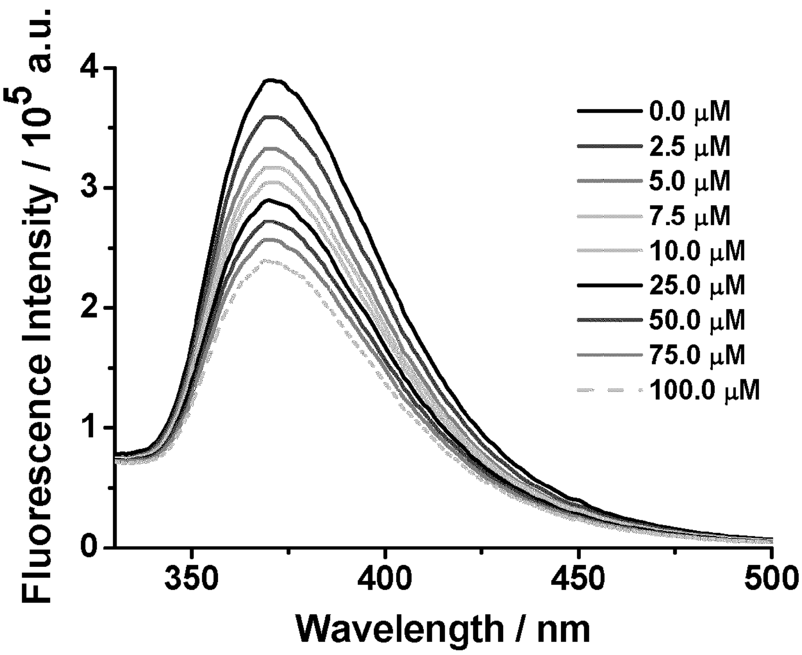
Fluorescence Intensity / $10^5$ a.u.
Wavelength / nm
0.0 μM
2.5 μM
5.0 μM
7.5 μM
10.0 μM
25.0 μM
50.0 μM
75.0 μM
100.0 μM

d)

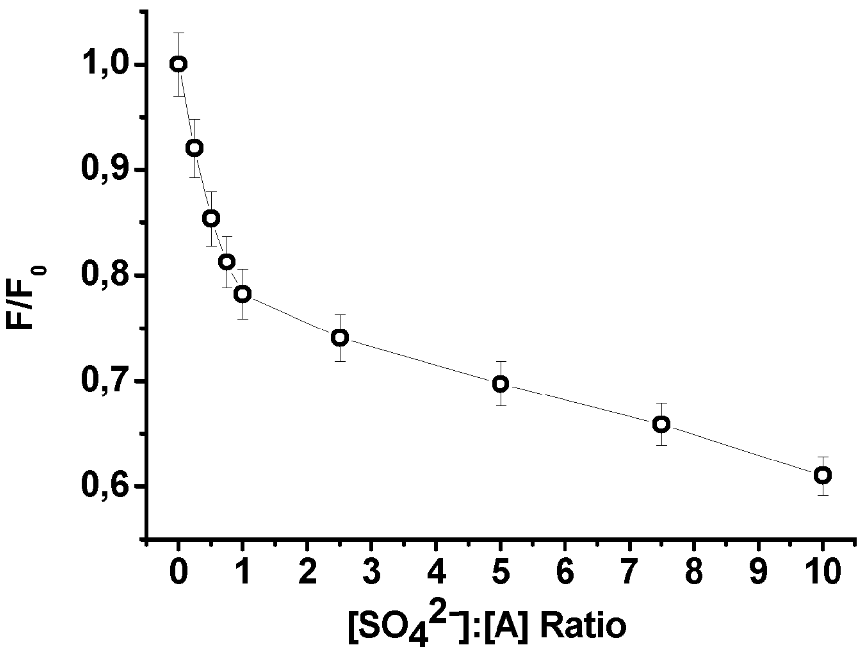
$F/F_0$
$[SO_4^{2-}]$:[A] Ratio

FIGURE 5

e)

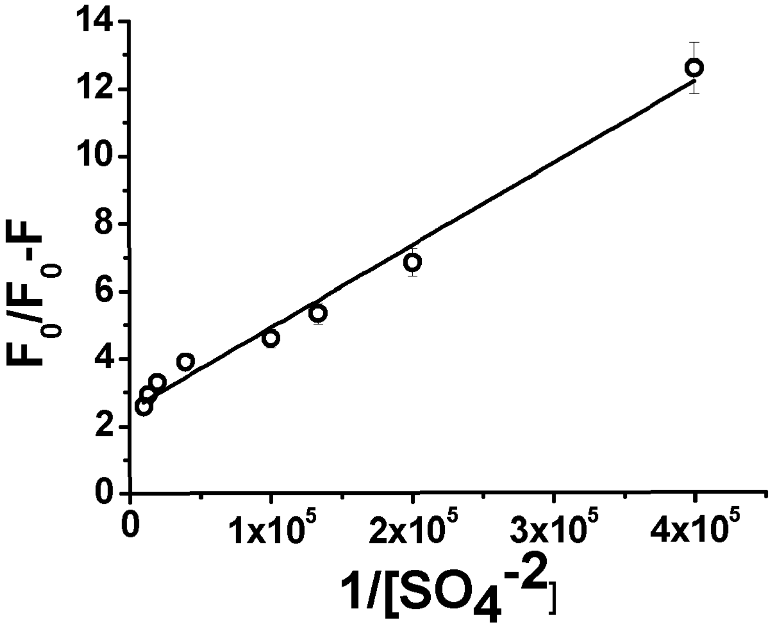

FIGURE 6

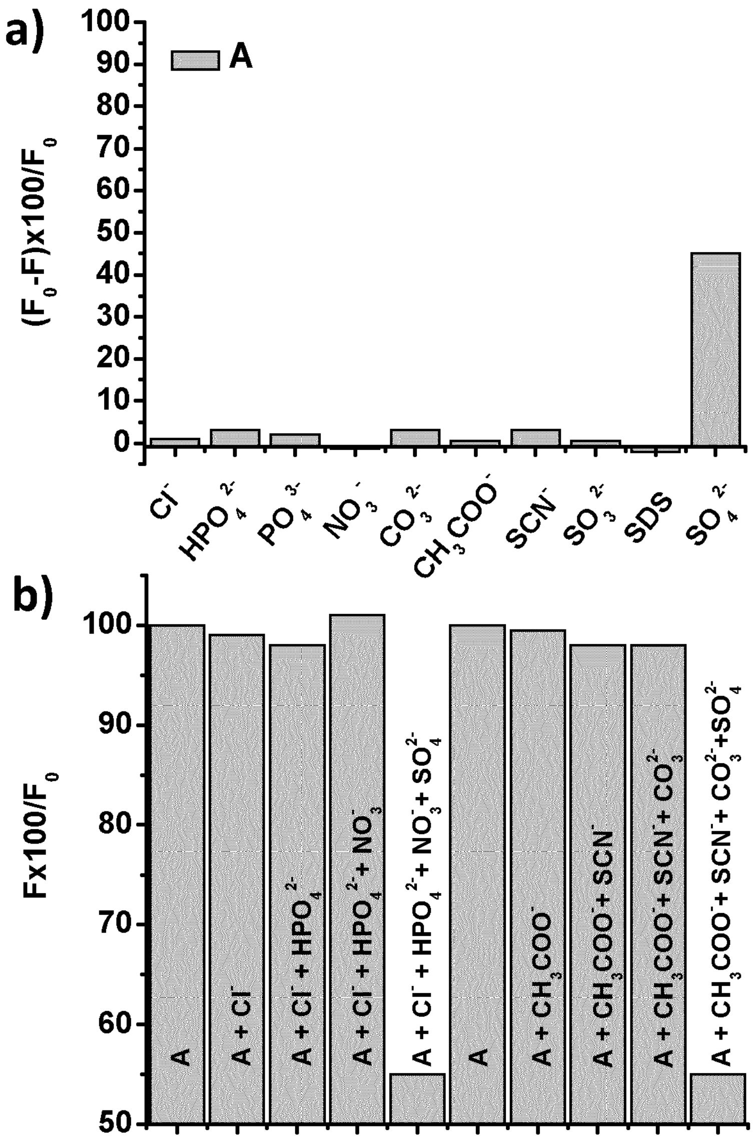
a)
A
(F0-F)x100/F0
100
90
80
70
60
50
40
30
20
10
0
Cl-
HPO4 2-
PO4 3-
NO3 -
CO3 2-
CH3COO-
SCN-
SO3 2-
SDS
SO4 2-
b)
Fx100/F0
100
90
80
70
60
50
A
A + Cl-
A + Cl- + HPO4 2-
A + Cl- + HPO4 2- + NO3-
A + Cl- + HPO4 2- + NO3- + SO4 2-
A
A + CH3COO-
A + CH3COO- + SCN-
A + CH3COO- + SCN- + CO3 2-
A + CH3COO- + SCN- + CO3 2- + SO4 2-

FIGURE 6

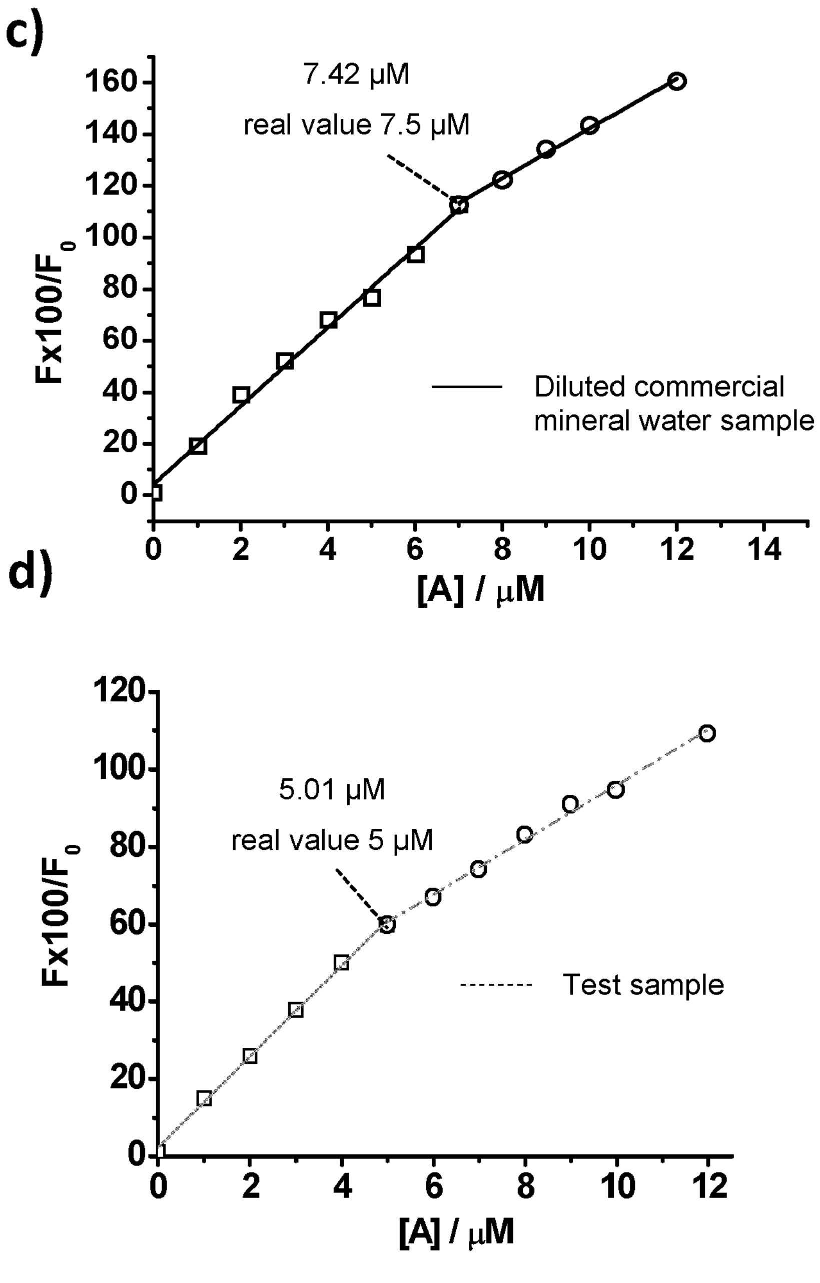
c)
7.42 µM
real value 7.5 µM
Diluted commercial mineral water sample
Fx100/F0
[A] / µM
d)
5.01 µM
real value 5 µM
Test sample
Fx100/F0
[A] / µM

FIGURE 7

a)

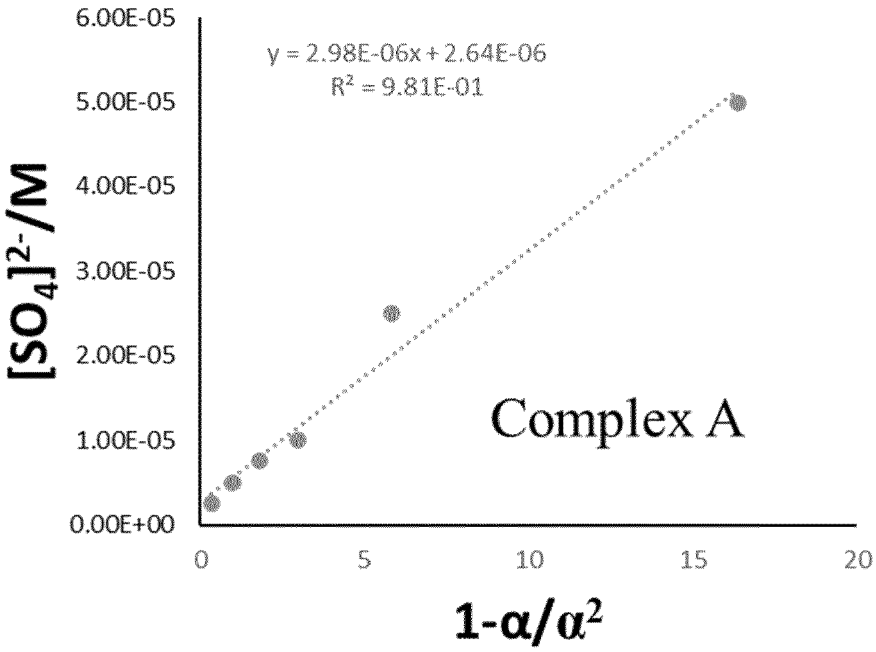
y = 2.98E-06x + 2.64E-06
R² = 9.81E-01
Complex A
[SO4]2-/M
1-α/α2
6.00E-05
5.00E-05
4.00E-05
3.00E-05
2.00E-05
1.00E-05
0.00E+00
0
5
10
15
20

b)

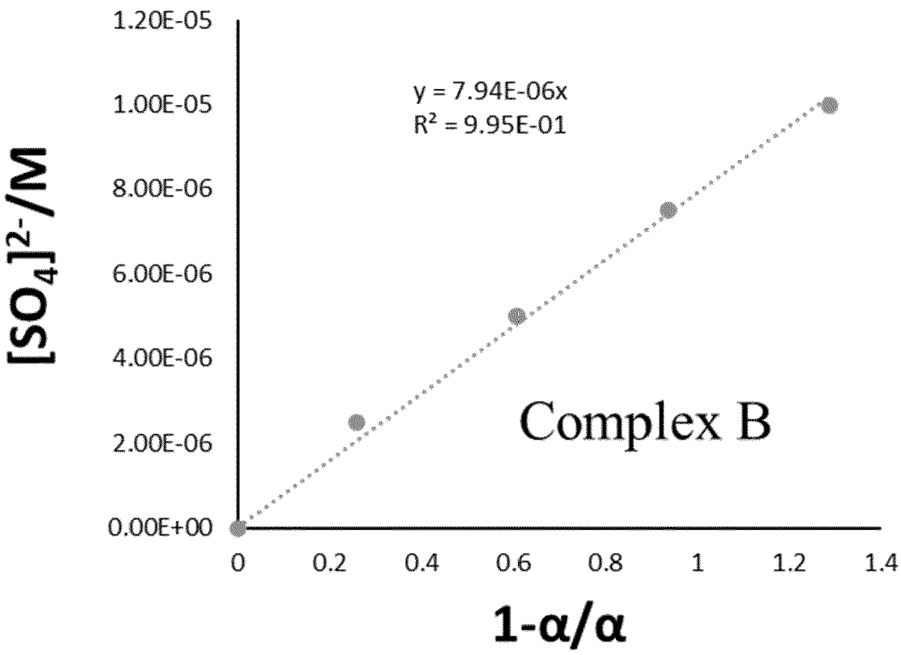
y = 7.94E-06x
R² = 9.95E-01
Complex B
[SO4]2-/M
1-α/α
1.20E-05
1.00E-05
8.00E-06
6.00E-06
4.00E-06
2.00E-06
0.00E+00
0
0.2
0.4
0.6
0.8
1
1.2
1.4

Europäisches Patentamt
European Patent Office
Office européen des brevets

# EUROPEAN SEARCH REPORT

Application Number
EP 21 38 2203

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | SAINI RAJNISH ET AL: "A fluorescent probe for the selective detection of sulfate ions in water", RSC ADVANCES, vol. 3, no. 44, 2013, page 21856, XP055835551, * figures 8,9 * | 1,3-9,15 | INV. G01N33/52 G01N33/84 |
| X | SHIBUTANI S ET AL: "Facile Aerial Oxidation of the DNA-Base Adduct N-(2'-Deoxyguanosin-8-yl)-2-aminofluorene [dG(C8)AF]", J. AM. CHEM. SOC., vol. 1124115, no. 4, 1990, pages 5667-5668, XP055835594, * compound 12 * | 2,10-14 | |
| X | KIM HEEMOON ET AL: "An anthracene appended guanidine derivative as water soluble fluorescence sensor for high pH values and water content measurements", JOURNAL OF PHOTOCHEMISTRY AND PHOTOBIOLOGY, A: CHEMISTRY, vol. 383, 5 August 2019 (2019-08-05), XP085785143, * Schemes 1-3 * | 2,10-14 | TECHNICAL FIELDS SEARCHED (IPC) G01N |
| X | WO 95/14467 A1 (CAMBRIDGE NEUROSCIENCE INC [US]; MAGAR SHARAD [US] ET AL.) 1 June 1995 (1995-06-01) * examples 13, 35 * | 2,10-14 | |
| X | WO 95/20950 A1 (CAMBRIDGE NEUROSCIENCE INC [US]; GOLDIN STANLEY M [US] ET AL.) 10 August 1995 (1995-08-10) * claim 13; examples 47, 52 * | 2,10-14 | |

-/--

The present search report has been drawn up for all claims

| Place of search | Date of completion of the search | Examiner |
|---|---|---|
| The Hague | 27 August 2021 | Gunster, Marco |

2

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 21 38 2203

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 01/83429 A1 (CHUGAI PHARMACEUTICAL CO LTD [JP]; HONDA TOSHIO [JP] ET AL.) 8 November 2001 (2001-11-08) * abstract; claim 1; compound 17b * ----- | 2,10-14 | |
| X | ARAFA REEM K. ET AL: "Synthesis, DNA Affinity, and Antiprotozoal Activity of Fused Ring Dicationic Compounds and Their Prodrugs", JOURNAL OF MEDICINAL CHEMISTRY, vol. 48, no. 17, 2005, pages 5480-5488, XP055835599, * compounds 12, 13 * ----- | 2,10-14 | |
| X | RODRIGUEZ FERNANDO ET AL: "Guanidine and 2-Aminoimidazoline Aromatic Derivatives as alpha2-Adrenoceptor Antagonists.2. Exploring Alkyl Linkers for New Antidepressants", JOURNAL OF MEDICINAL CHEMISTRY, vol. 51, 2008, pages 3304-3312, XP002534116, * compound 22b * ----- | 2,10-14 | TECHNICAL FIELDS SEARCHED (IPC) |
| X | NEHRBASS-STUEDLI ANGELA ET AL: "Novel diamidines with activity against Babesia devergens in vitro and Babesia microti in vivo", ANTIMICROBIAL AGENTS AND CHEMOTHERAPY, vol. 55, no. 7, 2 May 2011 (2011-05-02), pages 3439-3445, XP055835605, * page 3443; compound DB20C (13) * ----- | 2,10-14 | |

The present search report has been drawn up for all claims

2

| Place of search | Date of completion of the search | Examiner |
|---|---|---|
| The Hague | 27 August 2021 | Gunster, Marco |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

**ANNEX TO THE EUROPEAN SEARCH REPORT ON EUROPEAN PATENT APPLICATION NO.** EP 21 38 2203

This annex lists the patent family members relating to the patent documents cited in the above-mentioned European search report. The members are as contained in the European Patent Office EDP file on
The European Patent Office is in no way liable for these particulars which are merely given for the purpose of information.

27-08-2021

| Patent document cited in search report | | Publication date | Patent family member(s) | | Publication date |
|---|---|---|---|---|---|
| WO 9514467 | A1 | 01-06-1995 | AU | 703138 B2 | 18-03-1999 |
| | | | CA | 2177084 A1 | 01-06-1995 |
| | | | EP | 0746316 A1 | 11-12-1996 |
| | | | JP | H09505600 A | 03-06-1997 |
| | | | WO | 9514467 A1 | 01-06-1995 |
| | | | ZA | 949253 B | 04-01-1996 |
| WO 9520950 | A1 | 10-08-1995 | AU | 1912595 A | 21-08-1995 |
| | | | CA | 2182302 A1 | 10-08-1995 |
| | | | EP | 0751767 A1 | 08-01-1997 |
| | | | JP | H09509156 A | 16-09-1997 |
| | | | JP | 2007161725 A | 28-06-2007 |
| | | | KR | 100423272 B1 | 01-09-2004 |
| | | | US | 6174924 B1 | 16-01-2001 |
| | | | US | 6288123 B1 | 11-09-2001 |
| | | | US | 2007265348 A1 | 15-11-2007 |
| | | | WO | 9520950 A1 | 10-08-1995 |
| | | | ZA | 95878 B | 16-01-1996 |
| WO 0183429 | A1 | 08-11-2001 | AU | 5264801 A | 12-11-2001 |
| | | | WO | 0183429 A1 | 08-11-2001 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- DD 295919 A

### Non-patent literature cited in the description


- **Y. ZHANG ; P. S. CREMER.** *Annu. Rev. Phys. Chem.,* 2010, vol. 61, 63-83
- **I. RAVIKUMAR ; P. GHOSH.** *Chem. Soc. Rev.,* 2012, vol. 41, 3077
- **A. SINGH ; M. AGRAWAL.** *J. Environ. Biol.,* 2008, vol. 29, 15-24
- **L. C. BACKER.** *Crit. Rev. Clin. Lab. Sci.,* 2000, vol. 37, 389-400
- **P. A. GALE ; C. CALTAGIRONE.** *Coord. Chem. Rev.,* 2018, vol. 354, 2-27
- **K. ARIGA ; H. ITO ; J. P. HILL ; H. TSUKUBE.** *Chem. Soc. Rev.,* 2012, vol. 41, 58
- **H. ZHOU ; Y. ZHAO ; G. GAO ; S. LI ; J. LAN ; J. YOU.** *J. Am. Chem. Soc.,* 2013, vol. 135, 14908-14911
- **Q. LI ; Y. YUE ; Y. GUO ; S. SHAO.** *Sensors Actuators B Chem.,* 2012, vol. 173, 797-801
- **W. A. M. FERNANDO ; I.M.S.K. ILANKOON ; T. H. SYED ; M.YELLISHETTY.** *Minerals Engineering.,* 2018, vol. 117, 74-90
- **P. A. GALE.** *Chem. Commun.,* 2011, vol. 47, 82-86
- **L. CHEN ; S. N. BERRY ; X. WU ; E. N. W. HOWE ; P. A. GALE.** *Chem,* 2020, vol. 6, 61-141
- **M. J. LANGTON ; C. J. SERPELL ; P. D. BEER.** *Angew. Chemie Int. Ed.,* 2016, vol. 55, 1974-1987
- **C. MCDONAGH ; C. S. BURKE ; B. D. MACCRAITH.** *Chem. Rev.,* 2008, vol. 108, 400-422
- **S. KUBIK.** *Chem. Soc. Rev.,* 2010, vol. 39, 3648
- **E. A. KATAYEV ; Y. A. USTYNYUK ; J. L. SESSLER.** *Coord. Chem. Rev.,* 2006, vol. 250, 3004-3037
- **K. A. SCHUG ; W. LINDNER.** *Chem. Rev.,* 2005, vol. 105, 67-114
- **C. ALONSO-MORENO ; A. ANTIÑOLO ; F. CARRILLO-HERMOSILLA ; A. OTERO.** *Chem. Soc. Rev.,* 2014, vol. 43, 3406-3425
- **V. RAAB ; J. KIPKE ; R. M. GSCHWIND ; J. SUNDERMEYER.** *Chem. - A Eur. J.,* 2002, vol. 8, 1682-1693
- **J. RAN ; L. WU ; Y. HE ; Z. YANG ; Y. WANG ; C. JIANG ; L. GE ; E. BAKANGURA ; T. XU.** *J. Memb. Sci.,* 2017, vol. 522, 267-291
- **P. J. PACHECO-LIÑÁN ; I. BRAVO ; A. GARZÓN-RUIZ ; C. ALONSO-MORENO ; F. CARRILLO-HERMOSILLA ; A. ANTIÑOLO ; J. ALBALADEJO.** *J. Phys. Chem. C,* 2018, vol. 122, 9363-9373
- **E. BINDEWALD ; R. LORENZ ; O. HÜBNER ; D. BROX ; D.-P. HERTEN ; E. KAIFER ; H.-J. HIMMEL.** *Dalt. Trans.,* 2015, vol. 44, 3467-3485
- **P. J. PACHECO-LIÑÁN ; J. FERNANDEZ-SAINZ ; I. BRAVO ; A. GARZÓN-RUIZ ; C. ALONSO-MORENO ; F. CARRILLO-HERMOSILLA ; A. ANTIÑOLO ; J. ALBALADEJO.** *J. Phys. Chem. C,* 2018, vol. 122, 9363-9373
- **P. J. J. PACHECO LIÑÁN ; C. MARTIN ; C. ALONSO-MORENO ; A. JUAN ; D. HERMIDA MERINO ; A. GARZON ; J. ALBALADEJO ; M. VAN DER AUWERAER ; B. COHEN ; I. BRAVO.** *Chem. Commun.*
- **J. ZHOU ; H. LIU ; B. JIN ; X. LIU ; H. FU ; D. SHANGGUAN ; J. MATER.** *Chem. C,* 2013, vol. 1, 4427
- **M. P. COLES ; P. J. ARAGÓN-SÁEZ ; S. H. OAKLEY ; P. B. HITCHCOCK ; M. G. DAVIDSON ; Z. B. MAKSIC ; R. VIANELLO ; I. LEITO ; I. KALJURAND ; D. C. APPERLEY.** *J. Am. Chem. Soc.,* 2009, vol. 131, 16858-16868
- **K. KOBIRO ; Y. INOUE.** *J. Am. Chem. Soc.,* 2003, vol. 125, 421-427
- **ZHOU, H. ; ZHAO, Y. ; GAO, G. ; LI, S. ; LAN, J. ; YOU, J.** Highly Selective Fluorescent Recognition of Sulfate in Water by Two Rigid Tetrakisimidazolium Macrocycles with Peripheral Chains. *J. Am. Chem. Soc.,* 2013, vol. 135 (40), 14908-14911, https://doi.org/10.1021/ja406638b
- **YANG, W. ; YAN, J. ; FANG, H. ; WANG, B.** The First Fluorescent Sensor for D-Glucarate Based on the Cooperative Action of Boronic Acid and Guanidinium Groups. *Chem. Commun. (Cambridge, United Kingdom),* 2003, vol. 2 (6), 792-793